# EUROPEAN PATENT APPLICATION

(11) **EP 4 461 135 A1**
(43) Date of publication of application: **13.11.2024**
(21) Application number: 23382432.5
(22) Date of filing: 09.05.2023
(51) Int. Cl.: A01N 63/22, A01P 21/00

(54) **COMPOSITION COMPRISING BACILLUS SIAMENSIS STRAIN FOR PROMOTING PLANT GROWTH**

(71) Applicant: Bioiberica, S.A.U., 08389 Palafolls Barcelona (ES)
(72) Inventor: Velasco Álvarez, Javier, Palafolls (Barcelona) (ES); Sierras Serra, Nuria, Palafolls (Barcelona) (ES); Marín Garrido, Cándido, Palafolls (Barcelona) (ES)
(74) Representative: Pons

(57) **Abstract**

The present invention relates to the *Bacillus siamensis* strain CECT 30677 and to the composition comprising thereof, alone or in combination with, a fermentation broth and/or protein hydrolysate. Based on this, the invention provides a method for promoting, improving, stimulating or enhancing plant growth comprising applying to a plant, to a part thereof, or to the surroundings of the plant, said strain or composition.

## Description

The present invention relates to a strain of *Bacillus siamensis* deposited in the Spanish Type Culture Collection under the Budapest Treaty with deposit number CECT 30677, to the composition comprising said strain, and the use of both for promoting, improving, stimulating, or enhancing the plant growth or the plant yield. Thus, present invention relates to the agricultural field.

### BACKGROUND ART

The microflora surrounding plants is very diverse, including bacteria, fungi, yeast, algae. Some of these microorganisms may be deleterious to plants, and are often referred to as pathogens, while others may be beneficial to plants by promoting plant growth and crop productivity. Recent advances in soil microbiology and plant biotechnology have resulted in an increased interest in the use of microbial agents in agriculture, horticulture, forestry and environmental management. In particular, a number of microorganisms known to be present in soil ecological niche, generally known as rhizosphere and rhizoplane, have received considerable attention with respect to their ability to promote plant growth. Indeed, the rhizosphere soil represents a good reservoir of microbes for the potential isolation of beneficial microbes. Plant rhizosphere can contain billions of microorganisms in one gram of soil. In theory, microbial inoculants, without human intervention, have a low survival rate and efficacy in their natural soil environment because of the insufficient colony forming units per gram of soil. Therefore, since the 1960's, a number of biofertilizers that have an increased colony inoculum potential concentration have been developed and commercialized in an attempt to reduce the need for chemical synthetic fertilizers.

In addition, research conducted in recent years has shown that microorganisms can be used as biological control agents to increase agricultural productivity and efficiency. These studies have shown that various microorganisms are able to suppress plant pathogens and/or supplement plant growth, thus offering an attractive alternative to chemical pesticides with are less favoured because of their potentially negative impact on human health and environment quality.

Microorganisms which can stimulate plant growth are generally known as plant growth-promoting microbes (PGPM). In the past two decades, many PGPM species having positive influence on the growth of a wide variety of crop plants have been reported. PGPM are often universal symbionts of higher plants and are able to enhance the adaptive potential of their hosts through a number of mechanisms, such as the fixation of atmospheric nitrogen, the mobilization of recalcitrant soil nutrients (e.g., iron, phosphorous, sulfur etc.), the synthesis of phytohormones and vitamins, and the decomposition of plant materials in soils which often increases soil organic matter. Also, certain microbes can facilitate plant growth by controlling microbial species pathogenic to the plant (i.e., phytopathogens). For example, some beneficial microbes can control root rot in plants by competing with fungi for space on the surface of the plant root. In other instances, competition among various microbial strains in a plant's native microflora can stimulate root growth and increase the uptake of mineral nutrients and water to enhance plant yield. Therefore, biofertilizers can be developed as products based on microorganisms that naturally live in the soil. By increasing the population of beneficial microorganisms in the soil through inoculation, these soil microorganisms can boost their biological activity and, thus, supply the plants with important nutrients and beneficial factors that enhance their growth.

The inoculation of cultivated plants with PGPM is generally seen as a promising agricultural approach, for it allows pests to be controlled without using pesticides in large amounts. As environmental concerns about groundwater quality with excess fertilizer and pesticide exposure in foods grow, biological alternatives are becoming necessary. Thus, developing biological treatment compatible with fertilizers and pesticides or even reducing the amount of these chemical compounds could be a significant advancement in the agricultural industry. It has been established that stimulation of plant growth by PGPM is often closely related to the ability of the PGPM to colonize plant roots. However, relatively little attention has been given to the development of efficient selection procedures for obtaining microbial strains with high root-colonizing ability. The lack of such selection procedures slows down the study of plant-bacterial symbioses, and the deployment of PGPM in agriculture.

Therefore, there is a continuing need for the identification of new PGPM and/or testing of their compatibility with existing commercially available crop management products.

### DESCRIPTION OF THE INVENTION

The inventors have found that *Bacillus siamensis* strain PH-023 (also referred to as strain PH-023 or strain of the invention) has the ability to fix atmospheric nitrogen and produce siderophores and indole acetic acid (IAA), demonstrating the potential of this strain as a plant growth promoter and/or biostimulant.

Strain PH-23 was isolated from sediment from the mouth of the Vélez river. Analysis of the 16S rRNA gene identified this strain as *Bacillus siamensis* and it was deposited in the Spanish Type Culture Collection under the number CECT 30677. Analysis of the physiological and biochemical characteristics of this strain revealed that it is a large positive sporulating bacillus, which grows in a pH range between 5-10, is halotolerant, produces a wide variety of enzymes (amylases, proteases, alkaline phosphatase, acid phosphatase and nitrogenase), solubilises phosphate, uses atmospheric nitrogen as a nitrogen source, and produces siderophores and surfactants. Additional strain characteristics can be found in Examples 1 and 2.

Once the strain and its physiological and biochemical characteristics were identified, a series of *in vitro* assays were performed to demonstrate its plant growth promoting and/or biostimulant activity. These *in vitro* tests showed that strain PH-023 is able to fix environmental nitrogen, produce siderophores and IAA (see Example 3). Furthermore, analysis of the compounds present in the fermentation broth revealed the presence of metabolites with a biostimulant effect on plant growth as well as with biocontrol effects (Example 4). In addition, when the PH-023 strain is fermented using a hydrolysed protein, which is utilized as a carrier for final formulations, the abundance of secondary metabolites in the fermented broth of the strain increases in comparison when the bacteria is fermented employing an inorganic mineral salt as nitrogen source.

Additionally, *in vivo* assays in controlled conditions (greenhouse) and open field were carried out to demonstrate the plant growth promoting activity of strain PH-023. For this purpose, one formulation (named Product 1) based on (i) strain PH-023 formulated with (ii) its fermentation broth (obtained using protein hydrolysate as nitrogen source) and (iii) a protein hydrolysate (comprising the same composition that protein hydrolysate used during the fermentation process) as a carrier were developed and tested on pepper plants in comparison with Product 2 consisting only of the protein hydrolysate. From this trial it could be concluded that:
- a higher enzyme activity of the soil is showed with Product 1 allowing a higher nutrient solubilisation and assimilation by the crop and increasing the foliar content of essential macronutrients and micronutrients; and
- Both products 1 and 2 produce more fruits per plant, in relation to the control. However, Product 1 brings fruits of higher caliber in comparison with Product 2.

### Strain of the invention

Therefore, in one aspect, the present invention relates to a strain of *Bacillus siamensis* deposited in the Spanish Type Culture Collection under the Budapest Treaty with deposit number CECT 30677 (hereinafter "strain of the invention"), or a variant thereof having at least 99,5% of sequence identity with the 16S rRNA sequence of said strain comprising the sequence SEQ ID NO: 1.

The strain of the invention, also named *B. siamensis* PH-023 by the inventors, or just strain PH-023, was isolated from sediments at the mouth of Vélez river (Málaga, Spain). The strain was deposited by Bioibérica, S.A.U. on July 27, 2022 under the Budapest Treaty in the Spanish Type Culture Collection (CECT, calle Catedrático Agustín Escardino, 9, 46980 Paterna, Valencia, Spain). The assigned deposit number was CECT 30677.

The scientific classification of the strain of the invention is Domain: Bacteria; Phylum: Bacillota; Class: Bacilli; Order: Bacillates; Family: Bacillaceae; Genus: Bacillus; Species: *Bacillus siamensis.*

The strain of the invention is a Gram-positive, sporulated bacillus. It originates on solid media as white colonies with central elevation. Motile microorganism. It grows in a pH range between 5 - 10. It has no nutritional requirements. Halotolerant: grows between 0-15% NaCl.

It produces a wide variety of enzymes: amylases, proteases (caseinate and gelatinase), acid and alkaline phosphatase, and nitrogenase that allow it to use a wide variety of substrates as nutrients. It solubilizes phosphate (acid and alkaline phosphatase positive) and uses atmospheric nitrogen as a nitrogen source (Burk's medium growth positive). It produces siderophores. It does not hydrolyse chitin or cellulose, it does not produce HCN or SH₂, it has no lipase activity (the hydrolysis of Tween 80 and lecithin is negative). Produces 394 mg/L of surfactants (medium TSB).

The strain of the invention has one or more of the following properties: (i) plant disease protection activity, (ii) broad antifungal activity, (iii) broad antibacterial activity, and (iv) plant growth promoting activity or biostimulant. These properties make the strain of the invention capable of promoting, improving, stimulating, or enhancing the plant growth or the plant yield.

The present invention also encompasses a variant of the strain of the invention having at least 99.5%, 99.6%, 99,7%, 99.8%, 99,9% of sequence identity with the 16S rRNA sequence of said strain, wherein the capacities described throughout for the strain of the invention are retained, maintained or improved. The sequence of the 16S rRNA comprises the nucleotide sequence SEQ ID NO: 1.

SEQ ID NO: 1
ACCTGCAGGCGGCCGCACTAGTGATTAGAGTTTGATCATGGCTCAGGACGAACGCTGGCGGCGT
GCCTAATACATGCAAGTCGAGCGGACAGATGGGAGCTTGCTCCCTGATGTTAGCGGTGGACGGG
TGAGTAACACGTGGGTAACCTGCCTGTAAGACTGGGATAACTCCGGGAAACCGGGGCTAATACC
GGATGGTTGTCTGAACCGCATGGTTCAGACATAAAAGGTGGCTTCGGCTACCACTTACAGATGG
ACCCGCGGCGCATTAGCTAGTTGGTGAGGTAACGGCTCACCAAGGCGACGATGCGTAGCCGACC
TGAGAGGGTGATCGGCCACACTGGGACTGAGACACGGCCCAGACTCCTACGGGAGGCAGCAGTA
GGGAATCTTCCGCAATGGACGAAAGTCTGACGGAGCAACGCCGCGTGAGTGATGAAGGTTTTCG
GATCGTAAAGCTCCGTTGTTAGGGAAGAACAAGTGCCGTTCAAATAGGGCGGCACCTTGACGGT
ACCTAACCAGAAAGCCACGGCTAACTACGTGCCAGCAGCCGCGGTAATACGTAGGTGGCAAGCG
TTGTCCGGAATTATTGGGCGTAAAGGGCTCGCAGGCGGTTTCTTAAGTCTGATGTGAAAGCCCC
CGGCTCAACCGGGGAGGGTCATTGGAAACTGGGGAACTTGAGTGCAGAAGAGGAGAGTGGAATT
CCACGTGTAGCGGTGAAATGCGTAGAGATGTGGAGGAACACCAGTGGCGAAGGCGACTCTCTGG
TCTGTAACTGACGCTGAGGAGCGAAAGCGTGGGGAGCGAACAGGATTAGATACCCTGGTAGTCC
ACGCCGTAAACGATGAGTGCTAAGTGTTAGGGGGTTTCCTCCCCTTAGTGCTGCAGCTAACGCA
TTAAGCACTCCGCCTGGGGAGTACGGTCGCAAGACTGAAACTCAAAGGAATTGACGGGGGCCCG

CACAAGCGGTGGAGCATGTGGTTTAATTCGAAGCAACGCGAAGAACCTTACCAGGTCTTGACAT
CCTCTGACAATCCTAGAGATAGGACGTCCCCTTCGGGGGCAGAGTGACAGGTGGTGCATGGTTG
TCGTCAGCTCGTGTCGTGAGATGTTGGGTTAAGTCCCGCAACGAGCGCAACCCTTGATCTTAGT
TGCCAGCATTCAGTTGGGCACTCTAAGGTGACTGCCGGTGACAAACCGGAGGAAGGTGGGGATG
ACGTCAAATCATCATGCCCCTTATGACCTGGGCTACACACGTGCTACAATGGGCAGAACAAAGG
GCAGCGAAACCGCGAGGTTAAGCCAATCCCACAAATCTGTTCTCAGTTCGGATCGCGGTCTGCA
ACTCGACTGCGTGAAGCTGGAATCGCTAGTAATCGCGGATCAGCATGCCGCGGTGAATACGTTC
CCGGGCCTTGTACACACCGCCCGTCACACCACGAGAGTTTGTAACACCCGAAGTCGGTGAGGTA
ACCTTTTAGGAGCCAGCCGCCGAAGGTGGGACAGATGATTGGGGTGAAGTCCTAACAAGGTAAC
CGAATC

As used herein, the term "sequence identity" or "identity" is understood to mean the degree of similarity between two nucleotide or amino acid sequences obtained by aligning the two sequences. Depending on the number of common residues between the aligned sequences, a different degree of identity, expressed as a percentage, will be obtained. The degree of identity between two amino acid sequences may be determined by conventional methods, for example, by standard sequence alignment algorithms known in the state of the art, such as, for example, BLAST [Altschul S.F. et al. Basic local alignment search tool. J Mol Biol. 1990 Oct 5; 215(3): 403-10]. The BLAST programmes, for example, BLASTN, BLASTX, and TBLASTX, BLASTP and TBLASTN, are in the public domain at The National Center for Biotechnology Information (NCBI) website.

Persons skilled in the art understand that mutations in the nucleotide sequence of genes that lead to conservative amino acid substitutions at non-critical positions for the functionality of the protein are evolutionarily neutral mutations which do not affect its global structure or its functionality, giving rise to proteins which although comprise different amino acid sequence perform the same activity. Thus, strains having a 16S rRNA showing at least a 99.5% of sequence identity with the SEQ ID NO: 1 are considered "functionally equivalent variants" or "variants" of the strain of the invention. These "functionally equivalent variant" of the strain of the invention shows the same properties that the strain of the invention, i.e. (i) plant disease protection activity, (ii) broad antifungal activity, (iii) broad antibacterial activity, and (iv) plant growth promoting activity or biostimulant. These properties make the strain of the invention, and functionally equivalent variants thereof, capable of promoting, improving, or enhancing the plant growth or the plant yield. Examples of how to test if a strain given shows these properties are disclosed in the examples of the present description.

Thus, in a particular embodiment of the strain of the invention, the variant retains or further improves the activity of the strain of the invention in one or more of the following properties: (i) plant disease protection activity, (ii) broad antifungal activity, (iii) broad antibacterial activity, or (iv) plant growth promoting activity or biostimulant.

The variant can be produced naturally or intentionally by mutagenesis methods known in the state of the art such as, but not limited to, the growth of the original microorganism in the presence of mutagenic or stress-causing agents, or by genetic engineering aimed at modifying specific genes.

The strain *B. siamensis* CECT 30677, or any variant thereof, can be used in any way that exerts the described effects. Thus, according to a particular embodiment of the present invention, the strain *B. siamensis* CECT 30677 is in the form of viable cells (culturable or non-culturable) or in a sporulated form.

Another aspect of the present invention relates to a composition, hereinafter "composition of the invention", comprising
(i) the strain of the invention, and/or a variant thereof, and/or
(ii) the compounds and/or metabolites resulting from the fermentation of a protein hydrolysate by strain of the invention, or by a variant thereof.

The composition, defined in a general way, is a set of components made up of (i) at least the strain of the invention, and/or a variant thereof, in any concentration, and/or (ii) at least, the compounds and/or metabolites resulting from the fermentation of a protein hydrolysate by strain of the invention.

In another particular embodiment of the composition of the invention, alone or in combination with all or each one of the previous particular embodiments, the composition further comprises compounds and/or metabolites secreted by the strain of the invention or by a variant thereof. Examples of compounds and/or metabolites secreted by the strain of the invention, or by a variant thereof, include, without limiting to:
- Enzymes, such as amylase, proteases (caseinase, gelatinase etc.), acid phosphatases, alkaline phosphatases, nitrogenase, etc.
- Siderophores, such as Rhizobactin, Apoferrichrome, Straphyloferrin A, and Desferrioxamine.
- Surfactants, such as Surfactin.
- Phytohormones, such as precursor auxin (indol-3-acetamide), precursor of gibberellin A₄ 16,17-dihydro-16α,17-dihydroxy gibberellin A4, gibberellin A₄₃ and precursor of Jasmonate (methyl-jasmonate).
- Bactericides such as Bacicyclins.

In another particular embodiment of the composition of the invention, alone or in combination with each one or all the previous particular embodiments, the composition of the invention is a fermentation broth resulting from culturing the strain of the invention, or a variant thereof. In the present invention, the term "fermentation broth" or "whole broth" refers to the broth obtained by culturing the strain of the invention, or a variant thereof, in the proper conditions for the strain to carry out aerobic fermentation of the medium. Consequently, the fermentation broth comprises the fermentation products derived from the strain metabolism.

In another particular embodiment of the composition of the invention, alone or in combination with each one or all the previous particular embodiments, the medium used by the strain of the invention to carry out the fermentation may comprise a protein hydrolysate as nitrogen source. The inventors have found that when the strain of the invention uses a protein hydrolysate as nitrogen source during the fermentation, the abundance of secondary metabolites in the resulting fermented broth increases in comparison when the strain uses an inorganic mineral salt as nitrogen source.

As used herein, the term "protein hydrolysate" refers to a mixture of peptides of different chain length together with free amino acids prepared by splitting/hydrolyzing a protein with acid, alkali, or enzyme. The protein hydrolysate may be obtained from any source of protein, preferably, animal protein, such as meat from cow, sheep, goat, pig, etc., by enzymatic hydrolysis. Nevertheless, in a more particular embodiment of the composition of the invention, alone or in combination with each one or all the previous particular embodiments, the protein hydrolysate is obtained through an enzymatic hydrolysis from pig intestinal mucosa, more preferably, by enzymatic hydrolysis with a protease. The protein hydrolysate obtained through an enzymatic hydrolysis with a protease showed a degree of hydrolysis between 20% and 80%; moreover, it had a high protein content of peptides with an average molecular weight less than or equal 10,000 Daltons and a content of free amino acids between 2% and 5%.

In a particular embodiment of the composition of the invention, alone or in combination with all or each one of the previous particular embodiments, the compounds and/or metabolites resulting from the fermentation of the protein hydrolysate by the strain of the invention, or a variant thereof, comprises one or more of the following compounds: Dipeptide Glycil-phenylalanine, Indol-acetyl-phenylalanine, indole-3-acetamide, the inactivated form of Gibberellin A4, 16,17-dihydro-16α,17-dihydroxy gibberellin A4, Methyl-jasmonate, Pantothenic acid, a cyclic hexapeptide with the aminoacidic chain: cyclo-(Gly-Leu-Val-Ile-Ala-Phe), and/or siderophores.

In another particular embodiment of the composition of the invention, alone or in combination with all or each one of the previous particular embodiments, the concentration of the strain, or a variant thereof, in the composition of the invention is between 10⁵CFU/mL and 10¹² CFU/mL (both ends included).

In another particular embodiment of the composition of the invention, alone or in combination with each one or all the previous particular embodiments, the composition comprises a carrier. In the context of the present invention, the carrier is an agriculturally acceptable carrier. An "agriculturally acceptable carrier" or "agronomically acceptable carrier" means any material which can be used to deliver the actives (e.g., microorganism, agriculturally beneficial ingredient(s), biologically active ingredient(s), etc.) to a plant or a plant part (e.g., plant foliage) etc., and preferably which carrier can be applied (to the plant, plant part (e.g., foliage), or soil) without having an adverse effect on plant growth, soil structure, soil drainage or the like. Such carriers include, but are not limited to, protein hydrolysate, vermiculite, charcoal, sugar factory carbonation press mud, rice husk, carboxymethyl cellulose, peat, perlite, fine sand, calcium carbonate, flour, alum, a starch, talc, polyvinyl pyrrolidone, or a combination thereof. One of ordinary skill in the art can readily determine the appropriate carrier to be used taking into consideration factors such as the plant to which the composition of the invention is to be applied, type of soil, climate conditions, whether the composition is in liquid, solid or powder form, and the like. In a more particular embodiment, alone or in combination with each one or all the previous particular embodiments, the carrier is a liquid carrier or a solid carrier.

In another particular embodiment of the composition of the invention, alone or in combination with each one or all the previous particular embodiments, the agriculturally acceptable carrier used in the formulation of the composition of the invention is the same protein hydrolysate that the one used as nitrogen source in the fermentation process carried out by the strain of the invention, i.e. the carrier is the protein hydrolysate obtained from the enzymatic hydrolysis of pig intestinal mucosa with a protease

In view of the foregoing, in another particular embodiment of the composition of the invention, alone or in combination with the previous particular embodiment, the composition of the invention comprises
- the strain of the invention, or a variant thereof, and
- the fermentation broth resulting from the fermentation process by strain of the invention, or by a variant thereof, of a protein hydrolysate as nitrogen source, and
- the protein hydrolysate used in the fermentation process as nitrogen source.

As the skilled person in the art understand, the composition of the invention may comprise other kind of compounds usually used in agriculture. Thus, in another particular embodiment, alone or in combination with each one or all the previous particular embodiments, the composition further comprises at least one bioactive component, i.e. a component which can exert some positive effect in the growth of the plant, as long as said bioactive component is not detrimental to the strain of the invention. Examples of the bioactive components include, without limiting to, a pesticide, a fungicide, a herbicide, or a plant growth modifier, or a phytohormone. Consequently, in a more particular embodiment of the composition of the invention, alone or combination with each one or all of the previous particular embodiments, the composition of the invention comprises a bioactive component selected from the list consisting of a pesticide (such as fungicides, acaracides, molluskicides, insecticides, nematicides, etc), a fertilizer, a micronutrient fertilizer material, an herbicide, a plant growth regulator or any combination thereof.
- As used herein, the term "fungicide(s)" means any agent or combination of agents capable of killing fungi and/or inhibiting fungal growth. Examples of fungicides include, without limiting to, substituted benzene, a thiocarbamate, an ethylene bis dithiocarbamate, a thiophthalidamide, a copper compound, an organomercury compound, an organotin compound, a cadmium compound, anilazine, benomyl, cyclohexamide, dodine, etridiazole, iprodione, metlaxyl, thiamimefon, triforine, or a combination thereof.
- As used herein, the term "acaricide(s)" means any agent or combination of agents capable of killing one or more acarids and/or inhibiting the growth of one or more acarids. Examples of acaricides include, without limiting to, include chlorinated hydrocarbons (e.g., dichlorodiphenyltrichloroethane; DDT), organophosphorous compounds (e.g., Diazinon), carbamates (e.g., carbaryl), pyrethroids (e.g., permethrin, flumethrin), formamidines, and avermectins.
- As used herein, the term "molluskicides(s)" means any agent or combination of agents capable of killing one or more mollusc and/or inhibiting the growth of one or more mollusc, such as slugs and snails. Examples of molluskicides include, without limiting to, quanternary and polyquaternary ammonium compounds, aromatic hydrocarbons, endothall as the mono salt, metals like copper sulfate and their salts, niclosamide, chlorine, chlorine dioxide, chloramines, ozone, bromine, hydrogen peroxide and potassium permanganate.
- As used herein, the term "insecticide(s)" means any agent or combination of agents capable of killing one or more insects and/or inhibiting the growth of one or more insects. Examples of insecticides include, without limiting to, organophosphate, a carbamate, a pyrethroid, an acaricide, an alkyl phthalate, boric acid, a borate, a fluoride, sulfur, a haloaromatic substituted urea, a hydrocarbon ester, a biologically-based insecticide, or a combination thereof.
- As used herein, the term "nematicide(s)" means any agent or combination of agents capable of killing one or more nematodes and/or inhibiting the growth of one or more nematodes. Examples of nematicides include, without limiting to, 1,2-dichloropropane, 1,3-dichloropropene, chloropicrin, metam Sodium, Methyl Isothiocyanate (MITC), sodium tetrathiocarbonate, aldoxycarb, oxamyl, ethoprop, fenamiphos, fosthiazate
- Examples of fertilizers include, without limiting to, ammonium sulfate, ammonium nitrate, ammonium sulfate nitrate, ammonium chloride, ammonium bisulfate, ammonium polysulfide, ammonium thiosulfate, aqueous ammonia, anhydrous ammonia, ammonium polyphosphate, aluminum sulfate, calcium nitrate, calcium ammonium nitrate, calcium sulfate, calcined magnesite, calcitic limestone, calcium oxide, calcium nitrate, dolomitic limestone, hydrated lime, calcium carbonate, diammonium phosphate, monoammonium phosphate, magnesium nitrate, magnesium sulfate, potassium nitrate, potassium chloride, potassium magnesium sulfate, potassium sulfate, sodium nitrates, magnesian limestone, magnesia, urea, urea-formaldehydes, urea ammonium nitrate, sulfur- coated urea, polymer-coated urea, isobutylidene diurea, K2S04-2MgS04, kainite, sylvinite, kieserite, Epsom salts, elemental sulfur, marl, hydrolysed protein, ground oyster shells, fish meal, oil cakes, fish manure, blood meal, rock phosphate, super phosphates, slag, bone meal, wood ash, manure, bat guano, peat moss, compost, green sand, cottonseed meal, feather meal, crab meal, fish emulsion, or a combination thereof.
- Examples of micronutrient fertilizer materials include, without limiting to, boric acid, a borate, a boron frit, copper sulfate, a copper frit, a copper chelate, a sodium tetraborate decahydrate, an iron sulfate, an iron oxide, iron ammonium sulfate, an iron frit, an iron chelate, a manganese sulfate, a manganese oxide, a manganese chelate, a manganese chloride, a manganese frit, a sodium molybdate, molybdic acid, a zinc sulfate, a zinc oxide, a zinc carbonate, a zinc frit, zinc phosphate, a zinc chelate, or a combination thereof.
- As used herein, the term "herbicide(s)" means any agent or combination of agents capable of killing weeds and/or inhibiting the growth of weeds. Examples of herbicides include, without limiting to, chlorophenoxy compound, a nitrophenolic compound, a nitrocresolic compound, a dipyridyl compound, an acetamide, an aliphatic acid, an anilide, a benzamide, a benzoic acid, a benzoic acid derivitive, anisic acid, an anisic acid derivitive, a benzonitrile, benzothiadiazinone dioxide, a thiocarbamate, a carbamate, a carbanilate, chloropyridinyl, a cyclohexenone derivative, a dinitroaminobenzene derivative, a fluorodinitrotoluidine compound, isoxazolidinone, nicotinic acid, isopropylamine, an isopropylamine derivative, oxadiazolinone, a phosphate, a phthalate, a picolinic acid compound, a triazine, a triazole, a uracil, a urea derivative, endothall, sodium chlorate, the triazines (e.g., atrazine), the ureas, glyphosate, sulfosate, glyfosinate, sethoxydim, or a combination thereof.

- Examples of a plant growth regulators include, without limiting to, plant growth hormones such as at least one of the 84 identified gibberillins with GA3, GA4, GA5, CA7 and GA9 being preferred; cytokinins (e.g., zeatin, kinetin, benzyladenine, dihydrozeatin, and isopentenyl adenine); auxins (e.g., indolacetic acid (IAA), indolebutyric acid (IBA), and naphthalenacetic acid (NAA)); sodium ortho-nitrophenolate; sodium para-nitrophenolate; sodium 5-nitro-guaicolate; and polyhydroxycarboxylic acids of 2, 4, 5, and 6 carbon structures; ethephon; chlormequat chloride; mepiquat chloride.

In another particular embodiment of the composition of the invention, alone or in combination with each one or all the previous particular embodiments, the composition further comprises a surfactant and/or an additive.

Examples of surfactants include, without limiting to, a heavy petroleum oil, a heavy petroleum distillate, a polyol fatty acid ester, a polyethoxylated fatty acid ester, an aryl alkyl polyoxyethylene glycol, an alkyl amine acetate, an alkyl aryl sulfonate, a polyhydric alcohol, an alkyl phosphate, or a combination thereof.

Examples of additive include, without limiting to, an oil, a gum, a resin, a clay, a polyoxyethylene glycol, a terpene, a viscid organic, a fatty acid ester, a sulfated alcohol, an alkyl sulfonate, a petroleum sulfonate, an alcohol sulfate, a sodium alkyl butane diamate, a polyester of sodium thiobutant dioate, a benzene acetonitrile derivative, a proteinaceous material, or a combination thereof.

The composition may comprise other microorganisms further to the strain of the invention. Thus, in another particular embodiment, alone or in combination with each one or all the previous particular embodiments, the composition further comprises a microorganism different from the strain of the invention.

Examples of microorganisms different from the strain of the invention include, without limiting to, a bacterial inoculant of the genus Rhizobium, a bacterial inoculant of the genus Bradyrhizobium, Mesorhizobium, Azorhizobium, Allorhizobium, Sinorhizobium, Kluyvera, Azotobacter, Pseudomonas, Azospirillium, Bacillus, Streptomyces, Paenibacillus, Paracoccus, Enterobacter, Alcaligenes, Mycobacterium, Trichoderma, Gliocladium, Glomus, Klebsiella, or a combination thereof.

In a more particular embodiment of the composition of the invention, alone or in combination with each one or all of the previous particular embodiments, a microorganism different from the strain of the invention include, without limiting to, *Arthrobacter pascens, Arthrobacter ramosus, Arthrobacter oryzae, Arthrobacter humicola, Arthrobacter oxydans, Arthrobacter globiformis, Citricoccus alkalitolerans, Arthrobacter aurescens, Arthrobacter agilis, Arthrobacter tumbae, Arthrobacter arilaitensis, Arthrobacter crystallopoeietes, Kocuria rosea, Arthrobacter citreus, Georgenia ruanii, Microbacterium paludicola, Microbacterium aerolatum, Microbacterium testaceum, Microbacterium paraoxydans, Microbacterium phyllosphaerae, Microbacterium oxydans, Streptomyces griseus, Streptomyces olivoviridis, Streptomyces cirratus, Streptomyces candidus, Streptomyces chryseus, Streptomyces peucetius, Streptomyces flaveus, Streptomyces griseoruber, Streptomyces bottropensis, Streptomyces phaeochromogenes, Streptomyces fradeiae, Streptomyces durmitorensis, Streptomyces coeruleofuscus, Streptomyces marokkonensis, Streptomyces althioticus, Streptomyces pseudogriseolus, Streptomyces azureus, Williamensia muralis, Mycobacterium sacrum, Rhodococcus jostii, Nocardiopsis quinghaiensis, Terribacillus halophilus, Bacillus cibi, Bacillus idriensis, Bacillus licheniformis, Bacillus herbersteinensis, Bacillus simplex, Staphylococcus succinus, Bacillus benzoevorans, Bacillus niacin, Virgibacillus halodenitrificans, Oceanobacillus picturae, Bacillus psychordurans, Planomicrobium okeanokoites, Planococcus maritimus, Planococcus psychrotoleratus, Olivibacter soli, Bartonella elizabethae, Stenotrophomonas rhizophila, Halomonas aquamarina, Pseudomonas stutzeri, Pseudomonas fluorescens, Pseudomonas brassicacearum, Pseudomonas frederiksbergensis, Aspergillus fumigatiaffinis, Ochroconis sp., Micrococcus luteus, Citricoccus nitrophenolicus, Promicromonospora kroppenstedtii, Curtobacterium flaccumfaciens, Streptomyces halstedii, Paenibacillus tundrae, Exiguobacterium aurantiacum, Bacillus aquimaris, Planomicrobium koreense, Sinorhizobium medicae, Sinorhizobium meliloti, Mesorhizobium loti, Altererythrobacter luteolus, Massilia timonae, Stenotrophomonas maltophilia, Pseudomonas fulva, Pseudomonas syringae, Algoriphagus ratkowskyi, Phoma betae, Fusarium oxysporum, Cladosporium sphaerospermum, Alternaria thalictrigena, Fusarium equiseti, Penicillium chrysogenum, Penicillium commune, Pseudomonas putida, Pseudomonas plecoglossicida, Pseudomonas mosselii, Pseudomonas gessardii, Pseudomonas libanensis, Pseudomonas geniculate,* or any combination thereof.

The composition of the invention can be formulated in multiple ways. Suitable formulations include powders, especially wettable powders, soluble powders, solutions, suspension concentrate, granules, microparticles, seed treatments, liquid formulations, including suspensions of the compositions of the invention in water and encapsulations of bacteria.

The composition of the invention may be formulated in powders that are available in either a ready-to-use formulation or are mixed together at the time of use. The powder may be admixed with the soil prior to or at the time of planting. The composition of the invention may be formulated in a single, stable solution, or emulsion, or suspension. For solutions, the active chemical compounds are typically dissolved in solvents before the biological agent is added. Suitable liquid solvents include petroleum-based aromatics, such as xylene, toluene or alkylnaphthalenes, aliphatic hydrocarbons, such as cyclohexane or paraffins, for example petroleum fractions, mineral and vegetable oils, alcohols, such as butanol or glycol as well as their ethers and esters, ketones, such as methyl ethyl ketone, methyl isobutyl ketone or cyclohexanone, strongly polar solvents, such as dimethylformamide and dimethyl sulphoxide. For emulsion or suspension, the liquid medium is water. The chemical agent and biological agent are suspended in separate liquids and mixed at the time of application or are combined in a ready-to-use formulation that exhibits a reasonably long shelf-life. In use, the liquid can be applied through drip irrigation or sprayed or can be applied foliar as an atomized spray or in-furrow at the time of planting the crop. The liquid composition can be introduced in an effective amount on the seed (i.e., seed coating or dressing) or to the soil (i.e., in-furrow) before germination of the seed or directly to the soil in contact with the roots by utilizing a variety of techniques known in the art including, but not limited to, drip irrigation, sprinklers, soil injection or soil drenching.

One of ordinary skill in the art understands that an effective amount of the inventive compositions depends on the final formulation of the composition as well as the size of the plant or the size of the seed to be treated. Depending on the final formulation and method of application, one or more suitable additives can also be introduced to the compositions of the present invention.

Thus, in a particular embodiment of the composition of the invention, the composition is formulated as a granule, fine powder, wettable powder, dry flowables, microencapsulation of agents (microparticles), liquid formulation, solid formulation, whole broth culture, suspension concentrate or emulsifiable concentrate.

### Use of the strain of the invention.

As explained in the beginning of the present description, the strain of the invention shows a set of properties which make the strain of the invention capable of promoting, improving or enhancing the plant growth or the plant yield. Among these properties are (i) plant disease protection activity, (ii) broad antifungal activity, (iii) broad antibacterial activity, and (iv) plant growth promoting activity or biostimulant.

In view of the above, another aspect of the present invention relates to the use of the strain or the composition of the invention as defined above, hereinafter "use of the invention", for promoting, improving, stimulating, or enhancing plant growth.

In the context of the present invention, the terms "promoting", "improving", "stimulating", or "enhancing" are equivalent and can be used interchangeably throughout the present description.

The term "promoting plant growth" as used herein refers to the increase of one or more plant growth characteristics, to increase protein yield from the plant, or to increase grain yield of the plant, in comparison with a control plant, or parts thereof, that has not been inoculated with strain of the invention or as compared to a predetermined standard.

The one or more plant growth characteristics are selected from plant biomass (plant's height, weight, leaf size, root size, or stem size), plant growth rate, plant yield, root biomass, nodulation, nitrogen utilization, nutrient utilization, salt tolerance, resistance to one or more pathogens, resistance to fungal growth, growth under arid conditions, growth under arid soil conditions, growth under low pH conditions, growth under low pH soil conditions, growth under high pH conditions, growth under high pH soil conditions, growth under low temperature conditions, growth under low temperature soil conditions, growth under high temperature conditions, and growth under high temperature soil conditions.

The increase of plant growth achieved by the strain or composition of the invention can be measured and demonstrated in a number of ways. The increment of plant growth can be shown in instances wherein the average height of the plant is increased by at least about 5%, by at least about 10%, by at least about 15% or by at least about 20% as compared to the average height of plants grown under the same conditions but that have not been treated with the strain or composition of the invention. Also, the increment of plant growth can be shown in instances wherein the average leaf diameter of the leaves of plant is increased by at least about 5%, by at least about 10%, by at least about 15% or by at least about 20% as compared to the average leaf diameter of plants grown under the same conditions but that have not been treated with the strain or composition of the invention. Similarly, the increment of plant growth can be shown in instances wherein the average root length of the plant is increased by at least about 5%, by at least about 10%, by at least about 15% or by at least about 20% as compared to the average root length of the plants grown under the same conditions but that have not been treated with the strain or composition of the invention.

The strain or the composition can be used over any kind of plant. As used herein, the term "plant" refers to any living organism belonging to the kingdom Plantae (i.e., any genus/species in the Plant Kingdom). Likewise, the term "plant" encompasses plants that have been modified by one or more of breeding, mutagenesis and genetic engineering, as well as any plant propagules.

The plants with which the use of the invention may be practiced include, without limiting to, a dicotyledon plant, a monocotyledon plant or a gymnosperm plant.

Thus, in a particular embodiment of the use of the invention, alone or in combination with each one or all the previous particular embodiments, the plant is a dicotyledon plant, more particularly, the dicotyledon plant is selected from the group consisting of bean, pea, tomato, pepper, squash, alfalfa, almond, anise seed, apple, apricot, arracha, artichoke, avocado, bambara groundnut, beet, bergamot, black pepper, black wattle, blackberry, blueberry, bitter orange, bok-choi, Brazil nut, breadfruit, broccoli, broad bean, Brussels sprouts, buckwheat, cabbage, camelina, Chinese cabbage, cacao, cantaloupe, caraway seeds, cardoon, carob, carrot, cashew nuts, cassava, castor bean, cauliflower, celeriac, celery, cherry, chestnut, chickpea, chicory, chili pepper, chrysanthemum, cinnamon, citron, Clementine, clove, clover, coffee, cola nut, colza, corn for salad, cotton, cottonseed, cowpea, crambe, cranberry, cress, cucumber, currant, custard apple, drumstick tree, earth pea, eggplant, endive, fennel, fenugreek, fig, filbert, flax, geranium, gooseberry, gourd, grape, grapefruit, guava, hemp, hempseed, henna, hop, horse bean, horseradish, indigo, jasmine, Jerusalem artichoke, jute, kale, kapok, kenaf, kohlrabi, kumquat, lavender, lemon, lentil, lespedeza, lettuce, lime, liquorice, litchi, loquat, lupine, macadamia nut, mace, mandarin, mangel, mango, medlar, melon, mint, mulberry, mustard, nectarine, niger seed, nutmeg, okra, olive, opium, orange, papaya, parsnip, pea, peach, peanut, pear, pecan nut, persimmon, pigeon pea, pistachio nut, plantain, plum, pomegranate, pomelo, poppy seed, potato, sweet potato, prune, pumpkin, quebracho, quince, trees of the genus Cinchona, quinoa, radish, ramie, rapeseed, raspberry, rhea, rhubarb, rose, rubber, rutabaga, safflower, sainfoin, salsify, sapodilla, Satsuma, scorzonera, sesame, shea tree, soybean, spinach, squash, strawberry, sugar beet, sugarcane, sunflower, swede, sweet pepper, tangerine, tea, teff, tobacco, tomato, trefoil, tung tree, turnip, urena, vetch, walnut, watermelon, yerba mate, wintercress, shepherd's purse, garden cress, peppercress, watercress, pennycress, star anise, laurel, bay laurel, cassia, jamun, dill, tamarind, peppermint, oregano, rosemary, sage, soursop, pennywort, calophyllum, balsam pear, kukui nut, Tahitian chestnut, basil, huckleberry, hibiscus, passionfruit, star apple, sassafras, cactus, St. John's wort, loosestrife, hawthorn, cilantro, curry plant, kiwi, thyme, zucchini, ulluco, jicama, waterleaf, spiny monkey orange, yellow mombin, starfruit, amaranth, wasabi, Japanese pepper, yellow plum, mashua, Chinese toon, New Zealand spinach, bower spinach, ugu, tansy, chickweed, jocote, Malay apple, paracress, sowthistle, Chinese potato, horse parsley, hedge mustard, campion, agate, cassod tree, thistle, burnet, star gooseberry, saltwort, glasswort, sorrel, silver lace fern, collard greens, primrose, cowslip, purslane, knotgrass, terebinth, tree lettuce, wild betel, West African pepper, yerba santa, tarragon, parsley, chervil, land cress, burnet saxifrage, honeyherb, butterbur, shiso, water pepper, perilla, bitter bean, oca, kampong, Chinese celery, lemon basil, Thai basil, water mimosa, cicely, cabbage-tree, moringa, mauka, ostrich fern, rice paddy herb, yellow sawah lettuce, lovage, pepper grass, maca, bottle gourd, hyacinth bean, water spinach, catsear, fishwort, Okinawan spinach, lotus sweetjuice, gallant soldier, culantro, arugula, cardoon, caigua, mitsuba, chipilin, samphire, mampat, ebolo, ivy gourd, cabbage thistle, sea kale, chaya, huauzontle, Ethiopian mustard, magenta spreen, good king henry, epazole, lamb's quarters, centella plumed cockscomb, caper, rapini, napa cabbage, mizuna, Chinese savoy, kai-lan, mustard greens, Malabar spinach, chard, marshmallow, climbing wattle, China jute, paprika, annatto seed, spearmint, savory, marjoram, cumin, chamomile, lemon balm, allspice, bilberry, cherimoya, cloudberry, damson, pitaya, durian, elderberry, feijoa, jackfruit, jambul, jujube, physalis, purple mangosteen, rambutan, redcurrant, blackcurrant, salal berry, satsuma, ugli fruit, azuki bean, black bean, black-eyed pea, borlotti bean, common bean, green bean, kidney bean, lima bean, mung bean, navy bean, pinto bean, runner bean, mangetout, snap pea, broccoflower, calabrese, nettle, bell pepper, raddichio, daikon, white radish, skirret, tat soi, broccolini, black radish, burdock root, fava bean, broccoli raab, lablab, lupin, sterculia, velvet beans, winged beans, yam beans, mulga, ironweed, umbrella bush, tjuntjula, wakalpulka, witchetty bush, wiry wattle, chia, beech nut, candlenut, colocynth, mamoncillo, Maya nut, mongongo, ogbono nut, paradise nut, and cempedak.

In another particular embodiment of the use of the invention, alone or in combination with each one or all the previous particular embodiments, the plant is a monocotyledon plant, more particularly, the monocotyledon is selected from the group consisting of corn, wheat, oat, rice, barley, millet, banana, onion, garlic, asparagus, ryegrass, millet, fonio, raishan, nipa grass, turmeric, saffron, galangal, chive, cardamom, date palm, pineapple, shallot, leek, scallion, water chestnut, ramp, Job's tears, bamboo, ragi, spotless watermeal, arrowleaf elephant ear, Tahitian spinach, abaca, areca, bajra, betel nut, broom millet, broom sorghum, citronella, coconut, cocoyam, maize, dasheen, durra, durum wheat, edo, fique, formio, ginger, orchard grass, esparto grass, Sudan grass, guinea corn, Manila hemp, henequen, hybrid maize, jowar, lemon grass, maguey, bulrush millet, finger millet, foxtail millet, Japanese millet, proso millet, New Zealand flax, oats, oil palm, palm palmyra, sago palm, redtop, sisal, sorghum, spelt wheat, sweet corn, sweet sorghum, taro, teff, timothy grass, triticale, vanilla, wheat, and yam.

In another particular embodiment of the use of the invention, alone or in combination with each one or all the previous particular embodiments, the plant is a gymnosperm, more partially, the gymnosperm is from a family selected from the group consisting of Araucariaceae, Boweniaceae, Cephalotaxaceae, Cupressaceae, Cycadaceae, Ephedraceae, Ginkgoaceae, Gnetaceae, Pinaceae, Podocarpaceae, Taxaceae, Taxodiaceae, Welwitschiaceae, and Zamiaceae.

In a more particular embodiment of the use of the invention, alone or in combination with each one or all the previous particular embodiments, the plant is a horticultural crop, particularly, the horticultural crop is selected from the list consisting of vegetable, woody and field crops.

The term "horticultural crops" refers to any plant that is cultivated by people for food, for raw material, for medicinal purposes, and for aesthetic gratification. There are four major branches in horticultural crops: Olericulture which refers to vegetables; Pomology which refers to fruits; Floriculture which refers to flowers; and Landscaping which refers to garden plants. The strain or the composition of the invention can be used over any kind of these plants.

The term "vegetable crops" refers to plants which are mostly herbaceous annual plants of which some portion is eaten, either cooked or raw, during the principal part of the meal to complement starchy food and other food items. Illustrative, but not limiting examples of vegetable crops, include tomatoes, chilies, melons, watermelons, pepper, lettuce, spinach, potatoes, broccoli, carrots, etc.

The term "woody crops" refers to plants whose aerial part has a woody consistency and are non-rotational crops, other than permanent grassland, which occupy the soil for long periods of time and do not need to be transplanted after each harvest. The woody crops considered are: fruit trees, citrus fruit trees, nut trees, berry plantations, vineyards, olive trees and other permanent crops intended for human consumption (e.g. tea, coffee or carobs) and other purposes (e.g. nurseries, Christmas trees or plants for plaiting or weaving, such as rushes or bamboo). In a still more particular embodiment of the use of the invention, the woody crops are selected from the list consisting of fruit trees (seed and pome), olive groves, and vineyards.

The term "field crops" refers to any of the herbaceous plants grown on a large scale in cultivated fields: primarily a grain, forage, sugar, oil, or fiber crop.

More details about how to use the strain or the composition of the invention for promoting, improving or enhancing the plant growth, or the plant yield, are disclosed below for the method of the invention.

### Method of the invention

In another aspect, the present invention relates to a method for promoting, improving, stimulating, or enhancing plant growth, hereinafter "method of the invention", comprising applying to a plant, to a part thereof, or to the surroundings of the plant, an effective amount of (i) the strain of the invention or (ii) the composition of the invention.

The terms "promoting", "improving", "stimulating" or "enhancing" have been defined previously for the use of the invention and they are applicable to the present inventive aspect. Likewise, the term "plant" and the kind of plants to which the method of the invention can be applied (a dicotyledon, monocotyledon or gymnosperm plant) have also been defined and explained above.

As used herein, the term "plant part" refers to any part of a plant including but not limited to the shoot, root, stem, seeds, stipules, leaves, petals, flowers, ovules, bracts, branches, petioles, internodes, bark, wood, tubers, pubescence, tillers, rhizomes, fronds, blades, pollen, stamen, microspores, fruit and seed. The two main parts of plants grown in typical media employed in the art, such as soil, are often referred to as the "above-ground" part, also often referred to as the "shoots", and the "below-ground" part, also often referred to as the "roots".

The method of the invention comprises applying to a plant, to a part thereof, or to the surroundings of the plant, an effective amount of (i) the strain of the invention or (ii) the composition of the invention.

In the context of the present invention, the term "applying" refers to put into contact the strain or the composition of the invention with the plant, the part thereof, or the surroundings of the plant.

As it will be appreciated by those skilled in the art, the strain or the composition of the invention may be applied to the target plant using a variety of conventional methods such as dusting, coating, dressing, injecting, rubbing, rolling, dipping, spraying, or brushing, or any other appropriate technique which does not significantly injure the target plant to be treated. Other methods also include the inoculation of growth medium or soil with suspensions of the strain of the invention and the coating of plant seeds with the strain of the invention.

The strain or the composition of the invention can be applied by all methods of administration, such as spraying on stems and leaves, mixing into water supply, spraying on soil, injecting into subsoil using an injector, seed treatment including treatment of bulbs and tubers, and direct inoculation to plants.

In the case of application by mixing with feed water, for example, water is fed to the crop, or the water surface of a paddy field may be treated with granules or the like.

In the case of application by foliar application or application to soil, a planting hole or the vicinity thereof may be treated with granules or the like in the transplantation of seedling or the like, or seeds or the earth around a plant may be treated with granules, a wettable powder, or the like. In addition, it may be preferable to mix with soil after spraying on the soil. The amount of the active ingredient used for foliar application or application to the soil surface is, for example, from 0.5 to 5000 mg, and preferably from 3 to 3000 mg per 1 m² of agricultural and horticultural land.

In seed treatment, the agent is applied to the seeds by mixing and stirring wettable powders and dustable powders with the seeds or by dipping the seeds in diluted wettable powders. The seed treatment also includes seed coating treatments. The amount of active ingredients used in the case of seed treatment is, for example, from 0.005 to 10,000 g, and preferably from 0.05 to 1,000 g per 100 kg of the seeds. Seeds treated with agricultural or horticultural chemicals can be used in the same way as common seeds. Additionally, since the concentration and quantity used differ depending on the form of the agent, time of use, usage method, usage location, target crops and the like, they may be increased or decreased within the above ranges.

The compositions can be applied in an amount effective to enhance plant growth or yield relative to that in an untreated control. The active constituents are used in a concentration sufficient to enhance the growth of the target plant when applied to the plant. As will be apparent to a skilled person in the art, effective concentrations may vary depending upon various factors such as, for example, (a) the type of the plant or agricultural commodity; (b) the physiological condition of the plant or agricultural commodity; (c) the concentration of pathogens affecting the plant or agricultural commodity; (d) the type of disease injury on the plant or agricultural commodity; (e) weather conditions (e.g., temperature, humidity); and (f) the stage of plant disease. The skilled person in the art knows how to calculate the effective amount to apply depending on the above factors.

In a particular embodiment of the method of the invention, alone or in combination with each one or all the previous particular embodiments), the strain or the composition is applied to
- the roots, leaves, fruits, flowers, stems or seeds of the plant or to any combination thereof, and/or
- soil, compost, mulch, leaf litter, sawdust, straw, pine straw, wood chips, gravel, plant growing medium, other material in a bed surrounding the plant, or to any combination thereof.

### Kit of the invention and its use

The present invention also relates to kits for stimulating plant growth, which include the strain, or the composition of the invention as described herein, and instructions for applying it to a plant, to a part thereof, or to the surroundings of the plant. Therefore, in another aspect, the present invention relates to a kit, hereinafter "the kit of the invention", comprising the strain of the invention or the composition of the invention.

The kit of the invention will typically comprise one or more containers of the strain or composition of the invention as defined above in the present description, and printed instructions for using them for promoting plant growth. The kit can also include tools or instruments for reconstituting, measuring, mixing, or applying the strain or composition of the invention, and will vary in accordance with the particular formulation and intended use of the strain or composition of the invention.

In another aspect, the present invention relates to the use of the kit of the invention for for promoting, improving, stimulating, or enhancing plant growth or plant yield.

The terms "promoting", "improving", "stimulating" or "enhancing" have been defined previously for the use of the invention and they are applicable to the present inventive aspect. Likewise, the term "plant" and the kind of plants to which the method of the invention can be applied (a dicotyledon, monocotyledon or gymnosperm plant) have also been defined and explained above.

### Plant, or part thereof, of the invention

In another aspect, the present invention refers to a plant, or a part thereof, inoculated or coated with a strain of the invention, or with the composition of the invention. Hereinafter, "plant, or part thereof, of the invention".
the term "plant" or "part of a plant", and the kind of plants to which the present invention can be applied (a dicotyledon, monocotyledon or gymnosperm plant) have also been defined and explained above.

In the context of the present invention, it is contemplated that the plants or part thereof, such as seeds, can be substantially uniformly coated with one or more layers of the strain or composition of the invention as disclosed herein using conventional methods of mixing, spraying or a combination thereof through the use of treatment application equipment that is specifically designed and manufactured to accurately, safely, and efficiently apply products to plants or part thereof, particularly seeds. Such equipment uses various types of coating technology such as rotary coaters, drum coaters, fluidized bed techniques, spouted beds, rotary mists or a combination thereof. Liquid seed treatments can be applied via either a spinning "atomizer" disk or a spray nozzle which evenly distributes the seed treatment onto the seed as it moves though the spray pattern. Preferably, the seed is then mixed or tumbled for an additional period of time to achieve additional treatment distribution and drying. The seeds can be primed or unprimed before coating with the inventive compositions to increase the uniformity of germination and emergence. In an alternative embodiment, a dry powder formulation can be metered onto the moving seed and allowed to mix until completely distributed.

Alternatively, the plant or the part thereof may be partially coated with the strain or the composition of the invention, i.e. the plant or the part thereof, such as a seed, comprises at least part of the surface area coated with the strain or the composition of the invention.

In a particular embodiment of the plant, or part thereof, of the invention, the microorganism-treated the seeds have a microbial spore concentration or microbial cell concentration from about 10⁶ to about 10⁹ per seed. The seeds may also have more spores or microbial cells per seed, such as, for example 10¹⁰, 10¹¹ or 10¹² spores per seed. The microbial spores and/or cells can be coated freely onto the seeds or, preferably, they can be formulated in a liquid or solid composition before being coated onto the seeds. Seed coating methods and compositions that are known in the art can be particularly useful when they are modified by the addition of one of the embodiments of the present invention.

In some particular embodiments of the plant, or plant thereof, of the invention, alone or in combination with each one or all the previous particular embodiments, in addition to the microbial cells or spores, the coating can further comprise a layer of adherent. The adherent should be non-toxic, biodegradable, and adhesive. Examples of such materials include, but are not limited to, polyvinyl acetates; polyvinyl acetate copolymers; polyvinyl alcohols; polyvinyl alcohol copolymers; celluloses, such as methyl celluloses, hydroxymethyl celluloses, and hydroxymethyl propyl celluloses; dextrins; alginates; sugars; molasses; polyvinyl pyrrolidones; polysaccharides; proteins; fats; oils; gum arabics; gelatins; syrups; and starches.

In addition to the coating layer, the plant/part thereof, particularly the seed, may be treated with one or more of the following ingredients: other pesticides including fungicides and herbicides; herbicidal safeners; fertilizers and/or biocontrol agents. These compounds have been explained above and may be added as a separate layer or alternatively may be added in the coating layer.

In another particular embodiment of the plant/part thereof of the present invention, the strain of composition of the invention can be introduced onto a plant or part thereof, such as a seed, by use of solid matrix priming. For example, a quantity of the strain or composition of the invention can be mixed with a solid matrix material and then the plant or part thereof, such as a seed, can be placed into contact with the solid matrix material for a period to allow the strain or composition to be introduced to the plant or part thereof, such as a seed.

Then, the plant or part thereof, such as a seed, can optionally be separated from the solid matrix material and stored or used. Solid matrix materials which are useful in the present invention include polyacrylamide, starch, clay, silica, alumina, soil, sand, polyurea, polyacrylate, or any other material capable of absorbing or adsorbing the strain or the composition for a time and releasing that strain or composition into or onto the plant or part thereof, such as a seed. It is useful to make sure that the strain or composition of the invention and the solid matrix material are compatible with each other.

### BRIEF DESCRIPTION OF THE DRAWINGS

**Figure 1****.** Inoculation with PH-023 significantly increases the fresh weight of the plant.
**Figure 2****.** Effect of the strain of the invention in the number of nematodes eggs.
**Figure 3****.** Key biostimulant compounds, Indole-acetyl-phenylalanine precursor of Indole acetic acid. Comparison of starting fermentation broth (grey Box plot) with the final fermentation broth (black Box plot).
**Figure 4****.** Key biostimulant compounds, 16,17-dihidro-16α,17-dihydroxy gibberellin A4 precursor of Gibberellin A4. Comparison of starting fermentation broth (grey Box plot) with the final fermentation broth (black Box plot).
**Figure 5****.** Key biostimulant compounds, methyl-jasmonate precursor of Jasmonate. Comparison of starting fermentation broth (grey Box plot) with the final fermentation broth (black Box plot).
**Figure 6****.** Key biostimulant compounds, Vitamin B5 (Pantothenic acid). Comparison of starting fermentation broth (grey Box plot) with the final fermentation broth (black Box plot).
**Figure 7****.** Comparison of key siderophore, rhizobactin, abundance in two PH-023 broths using an inorganic N source (grey Box plot) and an organic N source (black Box plot).
**Figure 8****.** Comparison of key biostimulant compound, Indole-3-acetamide abundance in two PH-023 fermentation broths using an inorganic N source (grey Box plot) or an organic N source (black Box plot).
**Figure 9****.** Comparison of key biostimulant compound, Gibberellin A43 abundance in two PH-023 fermentation broths using an inorganic N source (grey Box plot) or an organic N source (black Box plot).
**Figure 10****.** Comparison of key biostimulant compound, Vitamin B5 (Pantothenic acid) abundance in two PH-023 fermentation broths using an inorganic N source (grey Box plot) or an organic N source (black Box plot).
**Figure 11****.** Comparison of key siderophore abundance in two PH-023 broths using an inorganic N source (grey Box plot) and an organic N source (black Box plot).
**Figure 12****.** *In vivo* tests in controlled conditions of the composition of the invention on pepper. Number of fruits per plant > 15 mm with Product 1 at 10 UHa and Product 2 at 20 UHa during first 09/09/2022 and second harvest 26/09/2022.
**Figure 13****.** Peach field trial. Percentage yield Tn /Ha. In the treatment with Product 1 at 20 L/Ha a significantly precocity in the harvest is obtained (+20% of production for the first cut) and a higher total production in comparison with the control. a, b: Means followed by same letter or symbol do not significantly differ (P=.05, Student-Newman-Keuls).
**Figure 14****.** Percentage of peach fruits by categories. The treatment with Product1, in an average, increases the two cuts of Category Extra of 73% and Category I with 18%. CAT Extra diameter fruits 67 mm ≤ 73 mm, CAT I 61 mm ≤ 67 mm, CAT II 56 mm ≤ 61 mm, 51 mm ≤ 56 mm.
**Figure 15****.** Pepper field trial. Number of fruits per plant and total number of fruits. In the treatment with Product 1 at 20 UHa, a precocity in the harvest is showed (+50% of fruits first cut) and a statistically higher total yield. a, b: Means followed by same letter or symbol do not significantly differ (P=0.05, Student-Newman-Keuls).
**Figure 16****.** Lettuce field trial. Product 1 shows a higher yield (10%) in comparison with the other two standard biofertilizers. Standard product 1 it is based on *B. velezencis* 1×10¹⁰ CFU/mL and 1% rhizobacteria and Standard product 2 consists of 12×10⁰⁷ CFU/mL *B. licheniformis, B. pumilus, B. safensis, B. velezensis.*
**Figure 17****.** Lettuce field trial. Product 1 shows a higher lettuce weight (10%) in comparison with the other two standard biofertilizers. Standard product 1 it is based on *B. velezencis* 1×10¹⁰ CFU/mL and 1% rhizobacteria and Standard product 2 comprises 12×10⁰⁷ CFU/mL *B. licheniformis, B. pumilus, B. safensis, B. velezensis.*

### EXAMPLES

Next, the invention will be illustrated by means of assays carried out by the inventors that demonstrate the effectiveness of the strain of the invention and aspect derived from it.

### EXAMPLE 1. Characterization of Bacillus siamensis PH-023 (strain of the invention)

### 1.1. Isolation and identification.

The strain was isolated from a sediment from the mouth of the river Vélez in Malaga (Spain). It was selected among hundreds of isolates for its surfactant activity.

For the identification of the strain, the 16S rRNA gene was amplified with universal primers (Forward primer, 16F27: 5'-AGAGTTTGATCATGGCTCAG-3' SEQ ID NO: 3; Reverse primer, 16R1488: 5'-CGGTTACCTTGTTAGGACTTCACC-3' SEQ ID NO: 4), and cloned into the pGEMT plasmid (Promega). Subsequently, this product was sequenced and a specific first intermediate for this strain was obtained. The 1542 bp sequence obtained indicated, when analyzed with the EZtaxon program (www.ezbioclud.net; Chun J, et al. 2007. Int J Syst Evol Microbiol. 57(Pt 10): 2259-2261. doi: 10.1099/ijs.0.64915-0. PMID: 17911292), an identity of 99.5% with the species B. *siamensis* and *B. velezensis.* To solve the identification, 1354 bp were analyzed, obviating the sequence of the ends of the gene. The results obtained indicate an identity of 99.6% with B. siamensis and 99.5% with *B. velezensis,* concluding that the strain PH-023 belongs to *Bacillus siamensis.* This strain, also named *B. siamensis* PH-023 by the inventors, or just strain PH-023, was deposited by Bioibérica, S.A.U. on July 27, 2022 under the Budapest Treaty in the Spanish Type Culture Collection. The assigned deposit number was CECT 30677.

The morphology of the strain corresponds to a Gram-positive motile sporulated Bacillus. It originates on solid media as white colonies with central elevation.

### 1.2. Physiological and biochemical characteristics

### 1.2.1. Growth.

It grows in a pH range between 5-10. It has no nutritional requirements. Halotolerant: grows between 0-15% NaCl.

### 1.2.2 Enzymes production.

It produces a wide variety of enzymes: amylases, proteases (caseinase and gelatinase), acid and alkaline phosphatase, and nitrogenase that allow it to use a wide variety of substrates as nutrients. It solubilizes phosphate (acid and alkaline phosphatase positive) and uses atmospheric nitrogen as a nitrogen source (Burk's medium growth positive). It produces siderophores (Table 1). It does not hydrolyze chitin or cellulose, it does not produce HCN or SH2, it has no lipase activity (the hydrolysis of Tween 80 and lecithin is negative).

**Table 1. Biochemical activities of the XB017 strain. Measurement: a) quantitative: indicative area of activity around the bacterial mass expressed in mm; b) qualitative: - no activity; +, ++, +++: low activity, medium activity, high activity.**

| | Measure |
|---|---|
| Growth in liquid and solid Burk medium | ++ |
| Acid phosphatase | + |
| Alkaline phosphatase | +++ |
| Hemolysis on blood agar | 5 |
| Starch hydrolysis | 7 |
| Casein hydrolysis | 6 |
| Gelatin liquefaction | ++ |
| Siderophore production | 4 |

### 1.2.3. Surfactant production.

The strain of the invention produces 394 mg/L of surfactants [medium TSB (*Tryptic Soy Broth*)]*,* and has activity against pathogenic bacteria such as *Agrobacterium tumefaciens, Pectobacterium atrosepticum* and *Ralstonia solanacearum* (Table 2). It was determined by placing the supernatant of the XB017 culture in a well-made in a TSA plate and seeding the pathogen on the surface. After incubation, the mm of the zone of inhibition of pathogen growth are determined.

**Table 2. Activity against pathogenic bacteria**

| | Measure |
|---|---|
| *R. solanacearum* | 3 |
| *P. atrosepticum* | 4 |
| *A. tumefaciens* | 6 |
| *Pectobacterium carotovorum* | - |
| *Xanthomonas campestris* | - |

### 1.2.4. Antifungal activity.

The antifungal activity of the strain of the invention was determined by two techniques: in solid medium and in liquid medium.

### ▪ Solid medium.

Using this technique, the percentage of inhibition of the growth of the fungal mycelium caused by bacterial growth is determined. It is done by seeding an extension of the PH-023 strain on a PDA plate and on the other end a portion of the fungal mycelium. After 15-21 days of incubation, the percentage of inhibition of the mycelium is calculated considering the maximum and minimum radius of growth of the fungus in the petri dish (Table 3).

**Table 3 - Antifungal activity of strain of the invention. *Mean of several determinations**

| | % inhibition of fungal mycelium* |
|---|---|
| *Alternaria alternata* | 33 |
| *Aspergillus niger* | 60 |
| *Botrytis cinerea* | 70 |
| *Fusarium oxysporum* | 20 |
| *Fusarium verticiloides* | 61 |
| *Monilinia fruticola* | 70 |
| *Phytophthora cactorum* | 78 |
| *Phytophthora cinamomi* | 50 |
| *Phytophthora capsici* | 66 |
| *Verticillium dahliae* | 55 |

### ▪ Liquid medium.

The ability of the strain of the invention (PH-023 strain) to prevent the germination of fungal spores was determined. For this determination, the strain was cultivated in a liquid medium for 72 hours and the supernatant was later collected by centrifugation. The fungus to be analyzed was cultivated for 15 days and then the mycelium was crushed and filtered through sterile gauze, adjusting to 10⁷ conidia/mL. In multiwell plates, 900 µL of the spore suspension of the pathogen was confronted with 300 µL of the supernatant of the PH-023 strain, incubating the sample for 15 days. The result was analyzed by observing the presence or absence of fungal growth. Positive and negative controls were used. The results obtained are shown in table 4.

**Table 4. Antifungal activity in liquid medium. (+) Antifungal activity: There is no fungus growth in the well; (-) Low or void antifungal activity: There is fungus growth.**

| | Antifungal activity |
|---|---|
| *Alternaria alternata* | - |
| *Botrytis cinerea* | + |
| *Fusarium oxysporum* | - |
| *Monilinia fruticola* | + |
| *Pvthium ultimum* | + |
| *Sclerotinia sclerotorium* | + |
| *Tanaterophorus cucumeris* | + |

As can be seen, the PH-023 strain has fungicidal activity against a large number of fungi: *A. niger, B. cinerea, F. verticilloides, M. fruticola, P. cactorum, P. capsici, P. ultimum, S. clerotorium* and *T. cucumeris.*

### 1.2.5. Plant growth promoting activity.

Seeds of susceptible tomato cv. Durinta F1 that were germinated in 25 mL capacity cells filled with sterile peat, kept in germination chambers at 25±2 ºC for 10 days. Bacterial inoculations were carried out four weeks after sowing and one week before transplanting, in the same germination trays. The bacterial suspensions were pipetted onto the lateral roots of the plants to be tested, at a dose of 2×10⁸ CFU per plant.

The tomato seedlings obtained were allowed to grow in a greenhouse at 25±2 ºC for another week, at which time they were transplanted into pots with 750 cm³ of sterile soil with or without the corresponding inoculum according to the treatments designed. Each treatment was replicated six times. The pots were distributed in the culture chamber in blocks, with the treatments randomly distributed within each block. A redistribution of the treatments was made every 7 days.

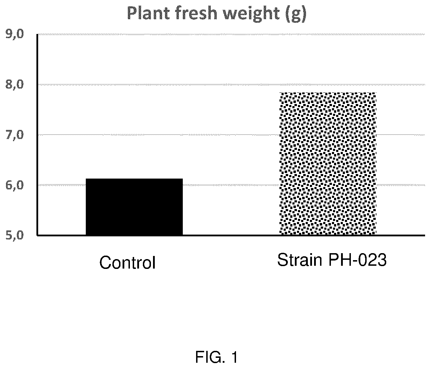

The tomato plants were allowed to grow in the culture chamber with an average soil temperature of 27±3 ºC. They were watered and fertilized according to the nutritional requirements of the plant, with a slow-release fertilizer (Osmocote Plus ^{®}: NPK 15 -10-12 + 2% MgO + microelements). The evaluation of the response of the plant was carried out 50 days after the transplant to the pots, by determining different parameters of plant growth. As can be seen in table 5 and Figure 1, inoculation with PH-023 significantly increases the fresh weight of the plant.

**Table 5. Tomato growth plant promoted by the strain of the invention.**

| Bacteria | Treatment | Repetition | FW stem | FW roots |
|---|---|---|---|---|
| 0 | 0-Ø | 1 | 6,55 | 1,50 |
| 0 | 0-Ø | 2 | 6,59 | 1,30 |
| 0 | 0-Ø | 3 | 6,41 | 1,50 |
| 0 | 0-Ø | 4 | 6,77 | 1,30 |
| 0 | 0-Ø | 5 | 6,94 | 2,40 |
| 0 | 0-Ø | 6 | 6,54 | 1,30 |
| PH-023 | PH-023-Ø | 1 | 8,91 | 3,40 |
| PH-023 | PH-023-Ø | 2 | 8,79 | 1,70 |
| PH-023 | PH-023-Ø | 3 | 8,51 | 1,90 |
| PH-023 | PH-023-Ø | 4 | 8,63 | 2,50 |
| PH-023 | PH-023-Ø | 5 | 9,02 | 1,50 |
| PH-023 | PH-023-Ø | 6 | 7,96 | 1,10 |

### 1.2.6. Activity against nematodes

Bacterial inoculum: A 48-h culture in liquid medium (TSB) was used.

Nematode inoculum: Suspensions of juveniles (J2s) of *Meloidogyne javanica* hatched from eggs were used. Eggs were extracted from susceptible tomato roots using the root maceration method, in a 0.5% w/v sodium hypochlorite solution (Hussey, R. S. and Barker, K. R., 1973, Plant Disease Reporter 57(12): 1025-1028). The eggs obtained hatched in Whitehead trays with a double cellulose paper filter (Whitehead, A. G. and Hemming, J. R., 1965. Annals of Applied Biology, 55, 25-38.).

In all cases, the following bioassay protocol was followed:
- Seeds of susceptible tomato cv. Durinta F1 were germinated in 25 ml capacity cells filled with sterile peat, kept in germination chambers at 25±2 °C for 10 days.
- The inoculations of the bacterial isolates were carried out four weeks after sowing and one week before transplanting, in the same germination trays. The bacterial suspensions were pipetted onto the lateral roots of the plants to be tested, at a dose of 2 × 10⁸ cfu per plant.
- The tomato seedlings obtained were allowed to grow in a greenhouse at 25±2 °C for another week, at which time they were transplanted into pots with 750 cm³ of sterile soil with or without the corresponding inoculum according to the treatments designed.
- 24 hours after transplanting, the plants were inoculated with aqueous suspensions of *M. javanica* J2, to obtain pot densities of 2 J2 per cm³ of soil. 3 mL of aqueous suspension with Meloidogyne juveniles (500 J2 per mL) were pipetted into three wells at 5 cm from the stem of the plant and to a depth of 5 cm, 1 mL per well. Each treatment was replicated six times. The pots were distributed in the culture chamber in blocks, with the treatments randomly distributed within each block. A redistribution of the treatments was made every 7 days.
- The tomato plants were allowed to grow in the culture chamber with an average soil temperature of 27±3 ºC. They were watered and fertilized according to the nutritional requirements of the plant, with a slow-release fertilizer (Osmocote Plus ^{®}: NPK 15 10-12 + 2% MgO + microelements).
- The evaluation of the response of the plant was carried out 50 days after the transplant to the pots, by determining the severity of the nodulation symptoms in the root system (Bridge, J. and Page, S.L.J., 1980. Tropical Pest Management, 26, 296-298), the reproduction rate of the nematodes (final population / initial population) and different plant growth parameters.
- Determination of nematode populations: Nematodes in soil were recovered from 250 cm³ samples by centrifugation in magnesium sulfate solutions. Nematodes were counted under a stereoscopic microscope with the aid of reticulated plates. All nematode instars (females, males, eggs, J2 and J3-J4) were counted independently. The average temperature during the test was 23.0 ± 1.4 °C

The values obtained in the six repetitions are indicated below (table 6):

**Table 6. Representation of mean values Rep. (Repetition). RI = Reproduction Index of the nematodes (final population/initial population).**

| | | | | Number of nematodes | | | | |
|---|---|---|---|---|---|---|---|---|
| Bacteria | Nematod e | Treatment | Rep . | 250 cm ³ soil | roots | Per g of root | pot | Rl |
| 0 | Ø | 0-Ø | 1 | 0 | 0 | 0 | 0 | 0,0 |
| 0 | Ø | 0-Ø | 2 | 0 | 0 | 0 | 0 | 0,0 |
| 0 | Ø | 0-Ø | 3 | 0 | 0 | 0 | 0 | 0,0 |
| 0 | Ø | 0-Ø | 4 | 0 | 0 | 0 | 0 | 0,0 |
| 0 | Ø | 0-Ø | 5 | 0 | 0 | 0 | 0 | 0,0 |
| 0 | Ø | 0-Ø | 6 | 0 | 0 | 0 | 0 | 0,0 |
| 0 | N | 0-N | 1 | 98 | 7300 | 6636 | 7594 | 5,1 |
| 0 | N | 0-N | 2 | 340 | 24500 | 7000 | 25520 | 17,0 |
| 0 | N | 0-N | 3 | 450 | 29600 | 18500 | 30950 | 20,6 |
| 0 | N | 0-N | 4 | 142 | 12000 | 7500 | 12426 | 8,3 |
| 0 | N | 0-N | 5 | 650 | 32900 | 12185 | 34850 | 23,2 |
| 0 | N | 0-N | 6 | 157 | 15100 | 10786 | 15571 | 10,4 |
| PH-023 | Ø | PH-023-Ø | 1 | 0 | 0 | 0 | 0 | 0,0 |
| PH-023 | Ø | PH-023-Ø | 2 | 0 | 0 | 0 | 0 | 0,0 |
| PH-023 | Ø | PH-023-Ø | 3 | 0 | 0 | 0 | 0 | 0,0 |
| PH-023 | Ø | PH-023-Ø | 4 | 0 | 0 | 0 | 0 | 0,0 |
| PH-023 | Ø | PH-023-Ø | 5 | 0 | 0 | 0 | 0 | 0,0 |
| PH-023 | Ø | PH-023-Ø | 6 | 0 | 0 | 0 | 0 | 0,0 |
| PH-023 | N | PH-023-N | 1 | 23 | 2200 | 1222 | 2269 | 1,5 |
| PH-023 | N | PH-023-N | 2 | 115 | 11800 | 8429 | 12145 | 8,1 |
| PH-023 | N | PH-023-N | 3 | 137 | 12000 | 9231 | 12411 | 8,3 |
| PH-023 | N | PH-023-N | 4 | 25 | 1450 | 967 | 1525 | 1,0 |
| PH-023 | N | PH-023-N | 5 | 83 | 6100 | 2542 | 6349 | 4,2 |
| PH-023 | N | PH-023-N | 6 | 28 | 2350 | 2156 | 2434 | 1,6 |

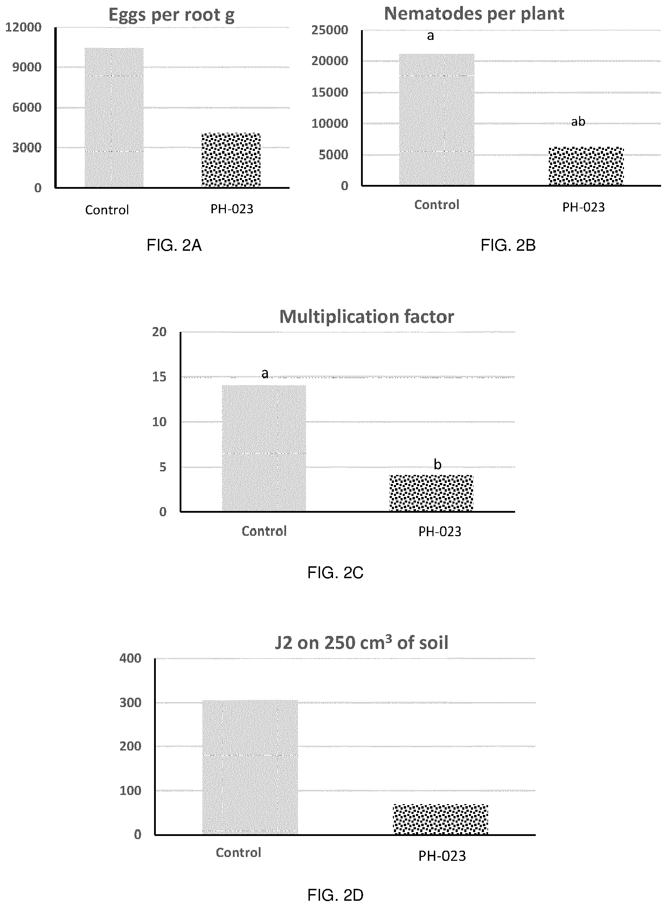

Figures 2A, 2B, 2C and 2D show the effect of the strain of the invention in the nematodes. The inoculation of PH-023 significantly reduced the number of nematodes eggs per root (Figure 2A), the number of nematodes per plant (Figure 2B), the multiplication rate of the nematodes (Figure 2C), and the number of nematodes per cm³ /soil (Figure 2D).

### 1.2.7. Activity against mealybug.

A 72-h culture of PH-023 was diluted in water in TSB (5×10⁸ CFU/mL) and applied 3 times with 7-day intervals to a lemon tree with mealybug. After time, its disappearance was observed.

### EXAMPLE 2. Growth of the strain of the invention in liquid medium. Manufacture of a fermentation broth.

The plate isolate of *Bacillus siamensis* PH-23 was transferred to a flask with a culture medium to generate the inoculum. Specifically, 5 mL cultured in SG medium (Leighton, T. J. and R. H. Doi. 1971. J. Biol. Chem. 246: 3189-3195.) for 48 hours, shaken at 30°C, is used as the initial inoculum.

These inoculums were seeded in 250 mL flasks containing 100 mL of medium and incubated under shaking (120 rpm) for 24 hours at 30°C. After this time, the content of each flask is sown in 2.5L flasks, containing 900 mL. It is kept under agitation for 5 days to sporulate. Next, in the bioreactor culture, a protein hydrolysate was used as a nitrogen source and technical glycerin as a carbon source. After approximately 72 hours, identification is carried out by visual method and counting to 10×10⁹ CFU/mL.

**Table 7. Culture medium composition: A) Protein hydrolysate Culture medium (protein hydrolysate and mineral salts); B) Mineral culture medium.**

| A) Protein hydrolysate Culture medium | | |
|---|---|---|
| Composition | per liter | Units |
| Glycerol | 20 | g |
| Corn steep liquor, CSL | 2 | g |
| Protein hydrolysate | 210 | mL |
| K2HPO4 | 4 | g |
| MgSO4 | 0,5 | g |
| MnSO4 | 0,1 | g |
| NaCl | 2 | g |
| CaCl2 | 0,02 | g |
| KOH | to pH 7 | |
| | | |

| B) Mineral culture medium | | |
|---|---|---|
| Composition | Per liter | Units |
| Glycerol | 20 | g |
| Corn steep liquor, CSL | 2 | g |
| (NH4)2SO4 | 1,3 | g |
| K2HPO4 | 4 | g |
| MgSO4 | 0,5 | g |
| MnSO4 | 0,1 | g |
| NaCl | 2 | g |
| CaCl2 | 0,02 | g |
| KOH | to pH 7 | |

The protein hydrolysate used as nitrogen source was obtained through the enzymatic hydrolysis with a protease of pig intestinal mucosa. This protein hydrolysate showed a degree of hydrolysis between 20% and 80% and had a high protein content of peptides with an average molecular weight less than or equal 10,000 Daltons and a content of free amino acids between 2% and 5%.

### EXAMPLE 3. In vitro study of the plant growth promoter activity of B. siamensis PH-023.

The present example shows the *in vitro* fertilizing and biostimulant effect of the B. *siamensis* strain PH-023, hereinafter strain PH-23 (strain of the invention). To determine the effect of the microorganism as a plant growth promoter (PGPM), a battery of parameters was carried out. The analyzes carried out consisted in the determination of the fixing capacity of environmental nitrogen, solubilization of forms of phosphorus and potassium; production of siderophores and the most important auxin, indoleacetic acid (IAA).

The results showed that strain PH-023, in addition to presenting atmospheric nitrogen fixation capacity, is also capable of producing siderophores, with a medium-low rate of production of these chelating compounds. In addition, the auxin analysis determined the production of 5.95 mg/mL of IAA. These results show the potential biostimulant and fertilizer effect of the strain PH-023.

### MATERIAL AND METHODS.

### Preparation of the microbial material.

The gram-positive bacterium *B. siamensis* strain PH-023 supplied in pure culture was evaluated. After receiving the species under study, the purity of the bacterium was determined by sowing by depletion of a colony and in a general medium for the growth of the bacterial strain, nutrient agar. After verifying the purity of the culture, the analyses began.

### Evaluated parameters.

The parameters were evaluated using differential and/or selective media for environmental nitrogen-fixing microorganisms, phosphorus, and potassium solubilizers, and siderophores producers. Auxin production was measured by spectrophotometry. The basics of each parameter are summarized below.

### Atmospheric nitrogen fixation

This test consists of the preparation of nitrogen-free medium (NFb), and where environmental nitrogen-fixing microorganisms can grow.

### Phosphorus solubilizers.

Pikovskaya medium was used to determine the phosphate solubilizing capacity, using Ca3(PO4)2 tricalcium phosphate as a phosphorus source. It is a quantitative determination of the solubilization of this macroelement that allows observing the solubilization through the production of a halo. The microorganisms that solubilize it do so through the production of organic acids, releasing phosphates and cations into the soil that are easily assimilated.

### Potassium solubilizers.

Potassium is one of the minerals with the highest requirements in plant nutrition. To carry out this analysis, two types of media were used, depending on the source of potassium provided to the differential medium.
▪ Method 1. Modified Pikovskaya medium (Oliveira et al, 2009. Soil Biology and Biochemistry, 41(9): 1782-1787). Using potassium nitrate (KNO3) as a source of potassium (K+), if the microorganism solubilizes K, the medium turns yellow, and a greater halo indicates greater element solubilization. This method evaluates the appearance of a solubilization halo (mm), bacterial growth (mm), solubilization index (IS), and relative solubilization efficiency (ERS).
   For the solubilization index, the formula A+B/A (A: colony diameter; B: halo diameter) was used (Kumar, V. and Narula, N. 1999. Biology and Fertility of Soils, 28, 301-305;). This is a quantitative parameter to evaluate the solubilization capacity of microorganisms. To determine the ERS, the formula (A/B)*100 (where A: diameter of the halo; B: diameter of the microorganism) was used (Ponmurugan, P. and Gopi, C. 2006. African Journal of biotechnology, 5.4: 348-350).
▪ Method 2. Medium Aleksandrov.
   The strains are grown in Aleksandrov medium, using calcium carbonate (CaCOs) as the only carbon source. Potassium feldspar is evaluated as a source of insoluble potassium. If the microorganism solubilizes K, a halo of precipitation is produced, indicative of the potential of the microorganism (the greater the halo, the greater the solubilizing potential).
   This method evaluates the appearance of solubilization halo (mm), bacterial growth (mm), solubilization index (IS), and relative solubilization efficiency (ERS). For the solubilization index, the formula A+B/A (A: colony diameter; B: halo diameter) was used (Kumar & Narula, 1999; Lara et al., 2011; cited above). This is a quantitative parameter to evaluate the solubilization capacity of microorganisms. To determine the ERS, the formula (A/B)*100 (where A: diameter of the halo; B: diameter of the microorganism) was used (Ponmurugan & Gopi, 2006, cited above).

### Siderophores production.

The production capacity of siderophores was evaluated using the universal CAS assay (chrome azurol s) in solid medium (Schwyn, B. and Neilands, J.B. 1987. Analytical biochemistry, 160.1: 47-56.). This method is the most widely used for the detection of siderophores due to the ferrichromogenic complex that changes color because of the loss of iron. Siderophore production was considered positive when observing a change from blue to yellow, orange or purple depending on the type of siderophore present (Pérez-Miranda, S. et al. 2007. J. Microbiol. Methods 70, 127-131).

### Auxins production.

Indoleacetic acid (IAA) produced by microorganisms is involved in promoting plant growth and root nodulation, intervening in the development of lateral roots (Lavenus, J. et al., 2013. Trends in plant science, 18.8: 450-458.), and increasing the number and length of absorbent hairs (Salazar-Henao, J. et al.; 2016 Development, 143.11: 1848-1858), which in turn allows better absorption of nutrients, helping its general growth (Aeron, A. et al.; 2011 Bacteria in agrobiology: crop ecosystems, 1-36). Auxin production is determined colorimetric (Khalid, A. et al., 2004 Journal of applied microbiology, 96.3: 473-480) by spectrophotometry and the units of measurement are mg/ml IAA.

### Assessment.

Nitrogen fixation capacity and potassium solubilization capacity (source: potassium nitrate) and siderophores production were evaluated for 7 days. The solubilization capacity of phosphates and potassium (potassium feldspar source) was evaluated for 14 days. Auxin production was evaluated after 96 hours of growth and constant agitation in tryptophan-free medium. Table 7 below shows the moments of evaluation of each of the parameters.

**Table 7. - Moments of evaluation.**

| **Parameter** | **Day 1** | **Day 2** | **Day 3** | **Day 4** | **Day 7** | **Day 14** |
|---|---|---|---|---|---|---|
| Atmospheric nitrogen fixation | x | x | x | x | x | |
| Phosphates solubilization. Source: tricalcium phosphate | x | x | x | x | x | x |
| Potassium solubilization. Potassium nitrate source | x | x | x | x | x | |
| Potassium solubilization. Feldspar potassium source | x | x | x | x | x | x |
| Siderophores production | x | x | x | x | x | |
| Auxin production: IAA | | | | x | | |

### Experimental design.

For the parameter's nitrogen fixation, phosphorus solubilizers, potassium solubilizers and siderophore producers, 4 replicates per strain were carried out; for auxin analysis, 3 replicates per strain were performed.

### RESULTS

### In vitro capacity for environmental nitrogen fixation

The results showed the nitrogen fixation capacity of the bacterial strain, observing that the strain PH-023 grew positively on this modified nitrogen-free (NFb) medium (Table 8).

**Table 8. Evolution of the environmental nitrogen fixation capacity of strain PH-023.**

| **Time** | **Nitrogen fixation** |
|---|---|
| 1 day | - |
| 2 days | + |
| 3 days | + |
| 4 days | + |
| 7 days | + |

### In vitro phosphate solubilization capacity.

Phosphorus solubilization capacity was determined by adding a disc impregnated with the pure microorganism in the center of a Petri dish. For 7 days, the appearance of the solubilization halo (mm), bacterial growth (mm), the solubilization index (IS) and the relative solubilization efficiency (ERS) were evaluated. The results showed the absence of solubilization of the strain, as shown in table 9. Referenced against the pull of analyzed microorganisms, 64% of the analyzed microorganisms lack the phosphorus solubilization capacity, this capacity being observed in fungi of the Pochonia genus, yeasts, or in bacteria of the Pseudomonas or Bacillus genera.

**Table 9. Evolution of the phosphorus solubilization capacity of the PH-023 strain**

| **Time** | **Solubilization capacity** | **Solubilization halo radius, mm** | **IS** | **%ERS** |
|---|---|---|---|---|
| 01 day | Absence | 0 | 0 | 0 |
| 02 days | Absence | 0 | 0 | 0 |
| 03 days | Absence | 0 | 0 | 0 |
| 04 days | Absence | 0 | 0 | 0 |
| 07 days | Absence | 0 | 0 | 0 |
| 14 days | Absence | 0 | 0 | 0 |

### In vitro potassium solubilization capacity.

Potassium solubilization capacity was determined by adding a disc impregnated with the pure microorganism in the center of a Petri dish of modified Pikovskaya medium and Aleksandrov medium. As in the previous section, the appearance of the solubilization halo was evaluated and measured as the radius of the halo (mm), the bacterial growth of the colony (mm), the solubilization index (IS) and the relative solubilization efficiency (ERS), these last two parameters were measured when the strain had the capacity to solubilize potassium. This strain lacks potassium solubilization mechanisms, both when the potassium source used was potassium nitrate and potassium feldspar (table 10).

**Table 10. Evolution of the potassium solubilization capacity of PH-023.**

| **Time** | **Solubilization capacity K** | **Radio halo de solubilizacion. mm** | **IS** | **%ERS** |
|---|---|---|---|---|
| 01 day | Absence | 0 | 0 | 0 |
| 02 days | Absence | 0 | 0 | 0 |
| 03 days | Absence | 0 | 0 | 0 |
| 04 days | Absence | 0 | 0 | 0 |
| 07 days | Absence | 0 | 0 | 0 |
| 14 days | Absence | 0 | 0 | 0 |

### Siderophores production capacity.

The production of siderophores is an indirect biological control mechanism since, as indicated by Shobha, G. and Kumudini, B. 2012. Int J Appl Sci Eng Res, 2012, 1.3: 463-474; Benite, A.N. and Machado, S. 2002. Química Nova, 25: 1155-1164; and Hotta, K. et al., 2010. Microbiology, 2010, 156.7: 1918-1925), this molecule chelates reduced iron present in the soil and deprives the pathogen that attacks the crop of this essential ion for its development. The production of siderophores in CAS medium was observed during 7 days of incubation. The production efficiency of these siderophores was evaluated by the siderophores production index, an index adapted from the existing one for the determination of solubilization efficiency. Thus, the IPS (siderophore production index) was evaluated by applying the following formula: A+B/A (A: colony diameter; B: halo diameter). Table 11 shows the positive production of siderophores from day 4, obtaining a maximum halo of 4.5 mm. The maximum production value of siderophores was 2.4. The decrease in IPS responds to a greater growth of the colony, having a greater production capacity of this molecule when after 4 days from its inoculation.

**Table 11. Siderophore production evolution.**

| Time | CAS-agar | Radio Halo Siderophore, mm | IPS |
|---|---|---|---|
| 1 day | - | 0.0 | 0.0 |
| 2 day | - | 0.0 | 0.0 |
| 3 day | - | 0.0 | 0.0 |
| 4 day | + | 4.0 | 2.4 |
| 7 day | + | 4.5 | 2.3 |

The production of siderophores is an important function and has a double effect; on the one hand they promote plant growth by increasing the availability of nutrients and on the other hand they can reduce the availability of iron for soil-borne plant pathogens (Chauhan, S. et al. 2012. Archives of Agronomy and Soil Science, 2012, 58.12: 1365-1385).

### Auxin, IAA production.

The results showed that the strain produced indole acetic acid, at a concentration of 5.95 mg/ml. (general average: 17.44 mg/mL).

### CONCLUSIONS

After the description of the materials and methods followed during the test, as well as the results obtained, the following conclusions can be established:
- Strain PH-023, in addition to presenting the ability to fix environmental nitrogen, can produce siderophores showing a medium-low index (IPS: 2.4) after 96 hours of incubation.
- The strain studied does not show mechanisms by which it solubilizes potassium or phosphates.
- The auxin analysis determined the production of 5.95 mg/mL of indole acetic acid. The production of this phytohormone is related to the regulation of plant development processes such as cell division, root formation and therefore its growth; so, its use in agriculture is very common.

### Example 4. Identification of the most abundant metabolites present in a microbial fermentation product at different stages.

### MATERIAL AND METHODS

### Fermentation conditions

The fermentation conditions are described in Example 2.

### Metabolomics analysis:

A. Sample preparation
   250 mg of product was extracted with methanol:water (80:20) at -20C (Sigma Aldrich) at -20ºC. Samples were shaken for 5 minutes at 4ºC with TissueLyser. Samples were centrifuged and filtered with cellulose filter of 0.22 µm. Method blanks and quality controls were included for blank noise subtraction and alignment purpose.
B. Chromatographic and mass spectrometric conditions for metabolomic RPLC-QTOF analysis (Bonini, P., et al.2020. Anal. Chem. 92, 7515-7522).
   The samples were injected at 8 µL and 10 µL for ESI positive and negative modes respectively, onto a Waters Acquity UPLC C18BEH maintained at 40°C was coupled to an Agilent 1290 Infinity UHPLC (Agilent Technologies). Data was processed by oloMAP 2.0 (Tsugawa, H. et al. 2020. Nat. Biotechnol. 38, 1159-1163).

### RESULTS

### 4.1. Identification of the most abundant secondary metabolites present in a microbial fermentation product at different stages that can have a plant biostimulant effect.

### Key biostimulants compounds.

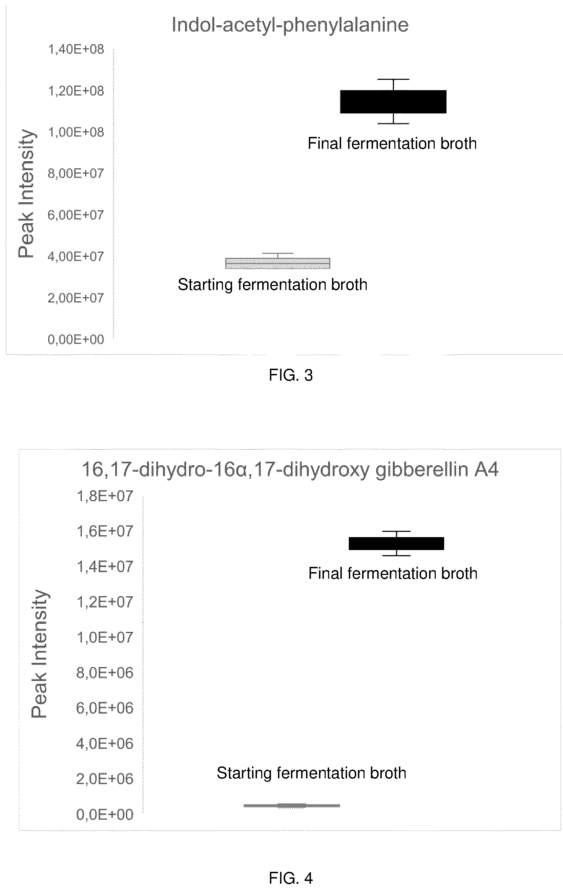

*Bacillus siamensis* PH-023 CECT 30677 can produce secondary metabolites related to Phenylalanine, such as the Dipeptide Glycil-phenylalanine and Indol-acetyl-phenylalanine. The second is particularly important because it is a storage form of Indoleacetic acid, a potent auxin hormone in plants. The reaction that forms this storage product is reversible, and it liberates the amino acid and the active auxin in the plant. In particular, in the auxin biosynthesis pathway, the precursor (Tryptophan) and an intermediate product (indol pyruvate) were detected in the fermentation broth, but we do not detect the end product as free indole acetic acid (Figure 3).

Regarding other type of plant biostimulant molecules, the inactivated form of Gibberellin A4 (one of the most active metabolites for stem elongation), 16,17-dihydro-16α,17-dihydroxy gibberellin A4, were found in the final fermentation broth (see Figure 4).

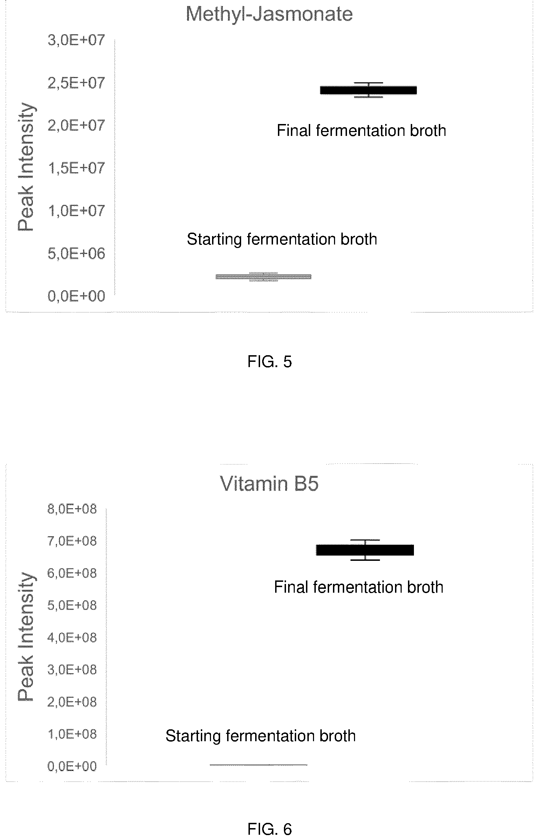

Finally, it was detected the direct precursor of Jasmonate (Methyl-jasmonate) in the final fermentation broth. The jasmonates are a group of important phytohormones, structurally like animal prostaglandins and ubiquitous in the plant kingdom. Many forms exist, based on the structure of Jasmonate (jasmonic acid, JA). The jasmonates act as regulatory molecules in many developmental processes that include fertility, sex determination, root elongation and fruit ripening. They are also potent signals activating plant defenses against pathogens, herbivory, wounding and abiotic stress. As can be seen in Figure 5, the amount of methyl-jasmonate is higher in the final fermentation broth in comparison with starting fermentation broth.

Vitamin B5 (Pantothenic acid) is also synthesized by the Bacteria object of this study. It is an interesting compound as is known for stimulating plant growth and nodulation of other microbial species on root plants. As can be seen in Figure 6, vitamin B5 is present in the final fermentation broth but absent in starting fermentation broth.

### 4.2. Identification of the most abundant metabolites present in a PH-23 fermentation product at different stages that can have a biocontrol effect against bacteria or fungi.

### Key Biocontrol compounds.

This particular Bacillus strain is able to produce antimicrobial compounds like Bacicyclin. In detail, it was detected 14 different variations of this class of molecule, a cyclic hexapeptide with this aminoacidic chain: cyclo-(Gly-Leu-Val-Ile-Ala-Phe). It also contains other type of antimicrobial compounds, mainly bactericide.

### 4.3. Identification and comparison of the most abundant secondary metabolites present in two microbial fermentations broths using different Nitrogen sources in the starting media composition.

The aim of the study was to analyse the differences in terms of secondary metabolites of two fermentation broths: the one obtained when PH-23 uses ammonium sulphate as a nitrogen source, and the one obtained when PH-23 uses a hydrolysed protein as nitrogen source.

### Key Biostimulans compounds.

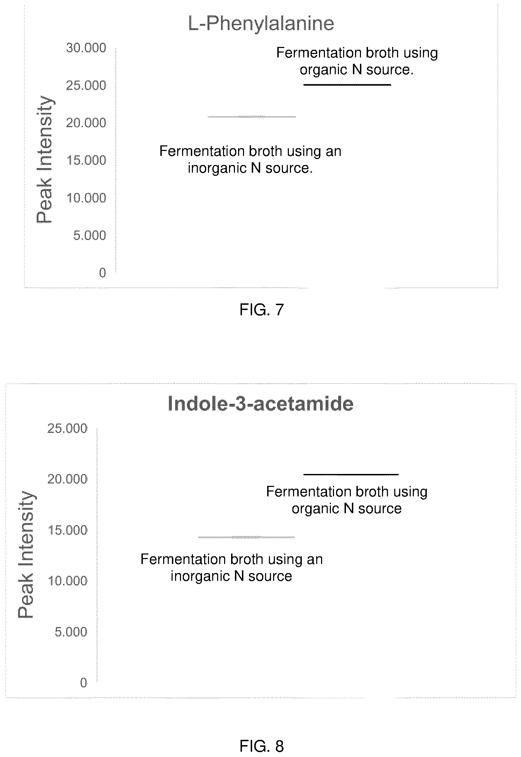

In the previous study (see point 4.2 above), it was found that the strain PH-23 of Bacillus has the metabolic pathway of the chorismite balanced in favour of the production of Phenylalanine. The high amount of this amino acid is then converted to Phenylacetaldehyde and Phenylethylamine. Comparing the amount of Phe from the two broths, it was found that when the hydrolysed protein is used as a nitrogen source for PH-023 strain fermentation, a higher concentration of this amino acid in the final broth is obtained (see Figure 7)

Interestingly, when the starting broth contains high number of amino acids, this does not trigger the synthesis by the bacteria, resulting in no changes from the beginning to the end. This, for example, is the case of Histidine. While the mineral broth has less, the bacteria have to synthesize it resulting in a higher concentration at the end of the process. In the carrier used as N source, this value does not change with the fermentation.

Metabolites detected in the previous study derived from Phenylalanine do not experience significant changes due to the different N-source. In this analysis, it was observed direct precursor of auxin (indole-3-acetamide) confirming that this Bacillus PH-23 is capable of its synthesis (Figure 8).

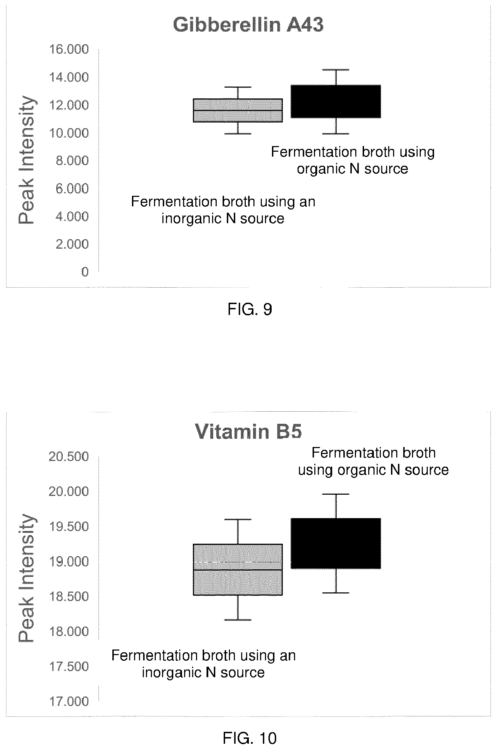

Regarding other type of plant biostimulant molecules, we found an active form of gibberellin A43 and vitamin B5 in both fermented products (Figure 9 and Figure 10 respectively).

### Key biocontrol compounds: Siderophore.

The bacterial strain object of the study was able to produce siderophores that are compounds able to bind iron. These compounds have the function to make unavailable iron for pathogen and improve plant nutrition.

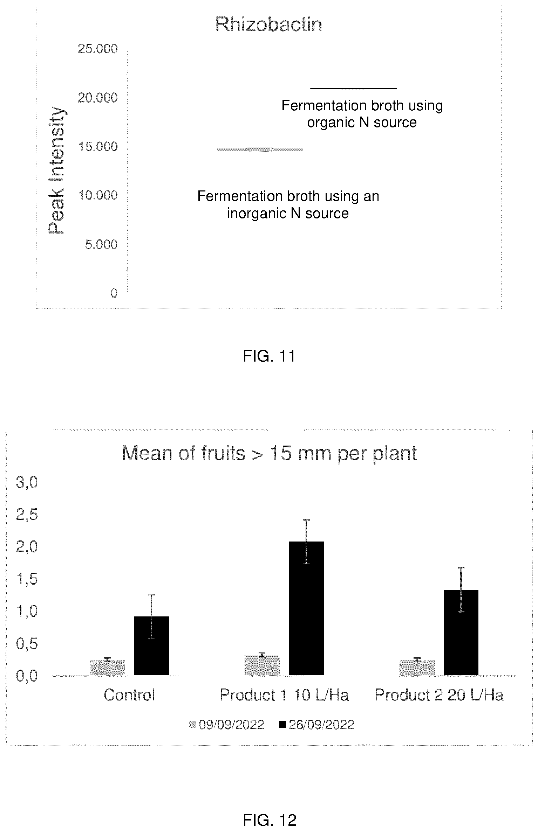

Regarding siderophores it was detected a metabolite that is highly abundant when the carrier is used as N-source: rhizobactin (Figure 11).

### Key biocontrol compounds: Bacicyclins.

As we found in the previous study (see point 4.2 above), this particular Bacillus strain PH-23 is able to produce antimicrobial compounds like Bacicyclin. In detail, it was detected several different variations of this class of molecule, a cyclic hexapeptide with this aminoacidic chain: cyclo-(Gly-Leu-Val-Ile-Ala-Phe) [SEQ ID NO: 2]. When the carrier is used as N-source, there is an increase from 2- to 5-fold the production of this class of metabolites in 7 cases, while it decreases in 4 cases. But, in general, there was an improvement in the synthesis of these compounds group.

### CONCLUSION

The use of a hydrolysed protein as a N-source increase the metabolic rate of the Bacillus PH-23 fermentation leading to a general increase of metabolism derived molecules with features as biostimulants and biocontrol.

### EXAMPLE 5. Manufacture of a composition comprising the strain of the invention.

### Formulation Product 1

Heat between 45 - 55 °C the protein hydrolysate and add a chelating solution, stabilizer solution and preservative and keep stirring for a minimum of 2 hours ± 15 minutes. Refrigerate the liquid until the temperature is below < 30°C and check that the temperature of the liquid to be filtered is below 30°C, if not, allow it to cool until it reaches this temperature. Filter the product through a layer of diatomaceous earth and adjust the pH between 4.9 - 5.5, if necessary, and keep stirring for a minimum of 20 minutes. Add the necessary quantity of the fermentation broth PH-023 as described in Example 2 above (Protein hydrolysate Culture medium) along with the strain of the invention and the hydrolysate protein as disclosed in Example 2 above.

### Formulation Product 2

Product 2 was formulated following the same method that Product 1 above, but without adding (i) the fermentation broth of PH-023 (as described in Example 2) above and (ii) the strain of the invention.

### EXAMPLE 6. In vivo assays in controlled conditions of the composition of the invention on pepper.

### MATERIAL AND METHODS

In controlled conditions (greenhouse), the efficiency of Product 1 at 10 L/Ha was assessed on pepper cv California in comparison with Product 2 at 20 L/Ha and the control (untreated plants). The study was carried with randomly distributed pots with 4 repetitions for each treatment consisting in three plants per replicate.

Three applications were made, the first between 10 days after transplant and the successive ones with an interval of 10 days.

### RESULTS

A trend towards a better global nutrient assimilation was observed, according to the foliar analysis results showed in Table 12 for both products in relation to the untreated one, although more favourable for Product 1 even when applied at a lower dose in comparison with Product 2..

**Table 12 - In vivo tests in controlled conditions of the composition of the invention on pepper. Pepper foliar analysis of macro and micronutrients for the untreated plants and treated with Product 1 and Product 2.**

| **% w/w** | **N** | **P** | **K** | **Mg** | **S** | **Fe (ppm)** | **Cu (ppm)** | **Mn (ppm)** | **Zn (ppm)** |
|---|---|---|---|---|---|---|---|---|---|
| **Control** | 2,77 | 0,18 | 6,14 | 1,01 | 0,49 | 135,13 | 5,83 | 62,34 | 52,86 |
| **Product 1** | 3,01 | 0,22 | 6,43 | 1,04 | 0,59 | 144,35 | 6,64 | 64,19 | 64,84 |
| **Product 2** | 2,74 | 0,19 | 6,16 | 1,07 | 0,56 | 148,05 | 6,06 | 64,01 | 73,1 |

Furthermore, Product 1 provides larger fruits on the second harvest, more fruits >15mm compared to Product 2 and the control (see Figure 12).

### EXAMPLE 7. Open field trial of the composition of the invention on peach.

### MATERIAL AND METHODS

The efficiency, in terms of quantity and quality on peach, of Product 1 at 20 L/Ha comparing with the untreated plot. The study was carried out with randomly distributed plots with 4 repetitions for each treatment experimental plot (replica) consisted of 20 trees of the UFO-4 variety.

Four applications were made by fertigation, the first between 5 and 15 days after full flowering and the successive ones with an interval of 12-14 days.

The applications were made 13 days after flowering (BBCH 67 growth stage) (Meier, Uwe, et al. Journal für Kulturpflanzen, 2009, 61.2: 41-52) and with an interval of 12 to 14 days through localized irrigation to the trees at dosage per hectare (20 UHa).

The homogeneity of variance (ANOVA) was verified for all treatments and evaluation dates using the Bartlett* test (Bartlett, M. S. 1937. Proceedings of the Royal Statistical Society, Series A 160, 268-282 JSTOR 96803).

To study the effect of the treatments applied for each evaluation date, the means were compared using the student-Newman-Keuls test (p=0.05) (Abdi, H.;Williams, L.J. Encycl. Res. Des. 2010, 2, 897-902).

### RESULTS

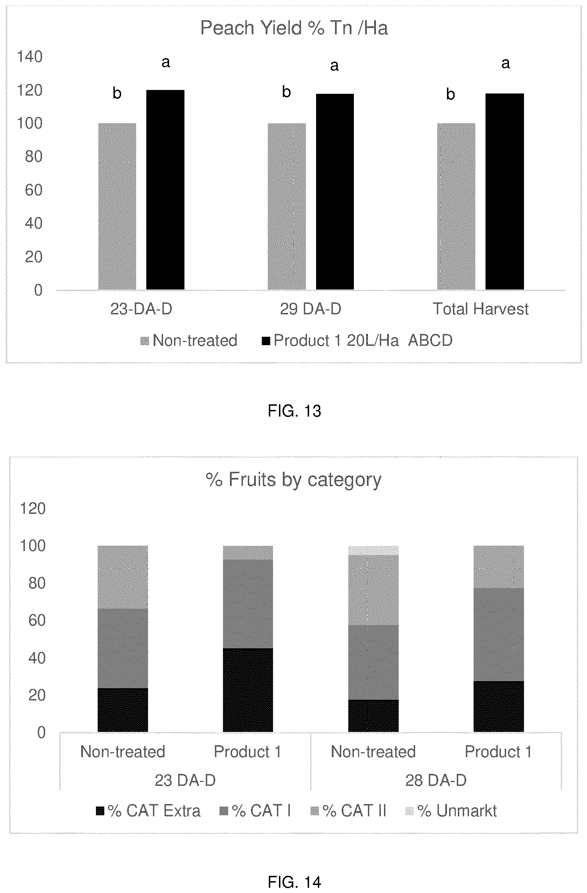

Statistically significant differences were obtained between the treatment with Product 1 at 20 Uha and the untreated for the harvest per hectare of the fruits in both harvests (23 DA-D and 28 DA-D) as well as with the accumulated yield with an increase of 18-20% over the control (Figure 13) which means a significantly higher precocity in the harvest and a higher total production.

Regarding quality, the treatment with Product 1, increases the two cuts of Category Extra with 73% and Category I with 18% (Figure 14)

### EXAMPLE 8. Open field trial of the composition of the invention on pepper.

### MATERIAL AND METHODS

The efficiency of Product 1 at 20 L/Ha, in terms of quantity and precocity in the harvest of pepper, was compared with a control. The study design was carried out using a randomly distributed plots with 4 repetitions for each treatment Each experimental plot (replica) consisted of 40 plants of Dulce Italian variety.

Four applications were made by fertigation, the first 20 days after the transplant and the successive ones with an interval of 10-13 days.

The harvests were carried out on days 30 DA-D, 37 DA-D and 44 DA-D.

The total number of fruits per plant was determined, as well as the accumulated.

The homogeneity of variance (ANOVA) was verified for all treatments and evaluation dates using the Bartlett* test.

To study the effect of the treatments applied for each evaluation date, the means were compared using the student-Newman-Keuls test (p=0.05).

### RESULTS

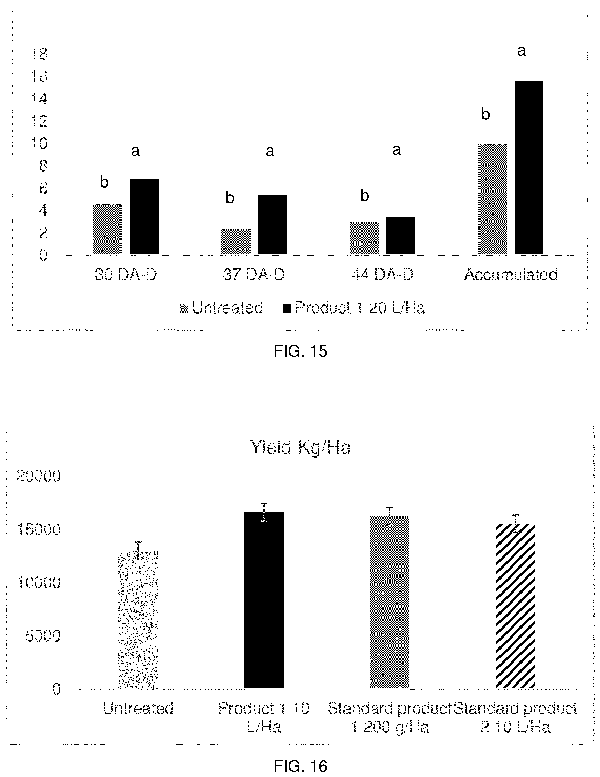

Statistically significant differences have been observed between the treatment with Product 1 and the control in all the harvests as well as with the accumulated number of fruits per plant (Figure 15).

### EXAMPLE 9. Open field trial of the composition of the invention on lettuce.

### MATERIAL AND METHODS

The efficiency of Product 1 at 10 L/Ha, in terms of quantity and quality in the harvest of lettuce, was compared with a control. The study design was carried out using a randomly distributed plots with 4 repetitions for each treatment Each experimental plot (replica) consisted of 45 plants of Baby Derby variety. Four applications were made. The first application 13 days after the transplant and the successive ones with an interval of seven days. Product 1 was compared with two standard biofertilizers, Standard product 1 based on *B. velezensis* 1×10¹⁰ CFU/mL and 1% rhizobacteria, and Standard product 2 based on 12×10⁰⁷ CFU/mL of *B. licheniformis, B. pumilus, B. safensis, B. velezensis.*

### RESULTS

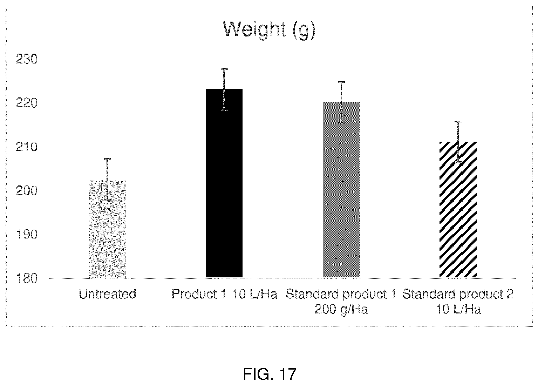

With Product 1 a higher yield is obtained (+28%) in relation to the Control Figure 16, with a higher lettuce weight (+10%) in relation to the rest of products Figure 17.

## Claims

1. A strain of *Bacillus siamensis* deposited in the Spanish Type Culture Collection under the Budapest Treaty with deposit number CECT 30677, or a variant thereof having at least 99.5% of sequence identity with the 16S rRNA sequence of said strain comprising the sequence SEQ ID NO: 1.

2. A strain according to claim 1, wherein the variant retains or further improves the activity of the strain *Bacillus siamensis* CECT 30677 in one or more of the following properties: (i) plant disease protection activity, (ii) broad antifungal activity, (iii) broad antibacterial activity, or (iv) plant growth promoting activity or biostimulant.

3. A strain according to claim 1 or 2, wherein the strain is a sporulated form.

4. A composition comprising a strain according to any one of claims 1 to 3, and/or the compounds and/or metabolites resulting from the fermentation of a protein hydrolysate by the strain according to any one of claims 1 to 3.

5. Composition according to claim 4, wherein compounds and/or metabolites resulting from the fermentation of a hydrolysed protein comprise one or more of the following compounds: Dipeptide Glycil-phenylalanine, Indol-acetyl-phenylalanine, indole-3-acetamide, the inactivated form of Gibberellin A4, 16,17-dihydro-16α,17-dihydroxy gibberellin A4, Methyl-jasmonate, Pantothenic acid, a cyclic hexapeptide with the aminoacidic chain cyclo-(Gly-Leu-Val-Ile-Ala-Phe) SEQ ID NO: 2, and/or siderophores.

6. Composition according to claim 4, wherein the composition further comprises a carrier, in particular, the carrier is a protein hydrolysate.

7. Composition according to any one of claims 4 to 6, wherein the composition further comprises
- a bioactive component, in particular, the bioactive component is selected from the list consisting of a pesticide, a fungicide, an herbicide, a plant growth modifier or a phytohormone, and/or
- a surfactant, and/or
- an additive; and/or
- a microorganism different from the strain according to any one of claims 1 to 3.

8. Composition according to any one of claims 4 to 7, wherein the concentration of the strain is between 10⁵CFU/mL and 10¹² CFU/mL.

9. Composition according to any one of claims 4 to 8, wherein the composition is formulated as a granule, fine powder, wettable powder, dry flowables, microencapsulation of agents, liquid formulation, solid formulation, whole broth culture, suspension concentrate or emulsifiable concentrate.

10. Use of a strain according to any one of claims 1 to 3, or a composition according to claims 4 to 9, for promoting, improving, stimulating or enhancing plant growth.

11. A method for promoting, improving, stimulating or enhancing plant growth comprising applying to a plant, to a part thereof, or to the surroundings of the plant, an effective amount of (i) a strain according to any one of claims 1 to 3, or (ii) a composition according to any one of claims 4 to 9.

12. Method according to claim 11, wherein the strain or the composition is applied to
- the roots, leaves, fruits, flowers, stems or seeds of the plant or to any combination thereof, and/or
- soil, compost, mulch, leaf litter, sawdust, straw, pine straw, wood chips, gravel, plant growing medium, other material in a bed surrounding the plant, or to any combination thereof.

13. Use according to claim 10 or a method according to claims 11 or 12, wherein the plant is a horticultural crop, particularly, the horticultural crop is selected from the list consisting of vegetable crops, woody crops and field crops, more particularly, the woody crops are selected from the list consisting of fruit trees, olive groves, and vineyards.

14. A kit comprising a strain according to any one of claims 1 to 3, or a composition according to claims 4 to 9.

15. Use of a kit according to claim 14, for promoting, improving, stimulating, or enhancing plant growth or plant yield.

16. A plant, or a part thereof, inoculated or coated with a strain according to any one of claims 1 to 3, or with a composition according to claims 4 to 9.
